# EUROPEAN PATENT SPECIFICATION

(11) **EP 3 249 710 B1**
(45) Date of publication and mention of the grant of the patent: **13.11.2019**
(21) Application number: 16740129.8
(22) Date of filing: 19.01.2016
(51) Int. Cl.: H01L 51/54, C07C 217/76

(54) **CHARGE-TRANSPORTING VARNISH, CHARGE-TRANSPORTING FILM, AND ORGANIC ELECTROLUMINESCENT ELEMENT**
LADUNGSTRANSPORTLACK, LADUNGSTRANSPORTFOLIE UND ORGANISCHES ELEKTROLUMINESZENZELEMENT
VERNIS DE TRANSPORT DE CHARGES, FILM DE TRANSPORT DE CHARGES ET ÉLÉMENT ÉLECTROLUMINESCENT ORGANIQUE

(30) Priority: 21.01.2015 JP 2015009497
(43) Date of publication of application: 29.11.2017
(73) Proprietor: Nissan Chemical Corporation, Tokyo (JP)
(72) Inventor: KOGA, Haruka, Funabashi-shi, Chiba 274-0052 (JP); TERAI, Seiya, Funabashi-shi, Chiba 274-0052 (JP)
(74) Representative: Mewburn Ellis LLP
(86) International application number: PCT/JP2016/051341
(87) International publication number: WO 2016/117522

(56) References cited:
- WO-A1-2010/058777
- WO-A1-2010/106806
- WO-A1-2015/162912
- CN-A- 102 219 700
- JP-A- 2013 234 169

## Description

### TECHNICAL FIELD

The present invention relates to a charge-transporting varnish, a charge-transporting thin film and an organic electroluminescent (EL) device.

### BACKGROUND ART

Charge-transporting thin films made of organic compounds are used as light-emitting layers or charge-injecting layers in organic EL devices. In particular, hole-injecting layers are responsible for transferring charge between an anode and a hole-transporting layer or a light-emitting layer, and thus serve an important function in achieving low-voltage driving and high brightness in organic EL devices.

Processes for forming hole-injecting layers are broadly divided into dry processes such as vapor deposition and wet processes such as spin coating. On comparing these types of processes, wet processes are better able to efficiently produce thin films having a high flatness over a large surface area. Therefore, as organic EL displays of increasingly large surface area are being developed, there exists a desire today for hole-injecting layers that can be formed by wet processes.

In light of such circumstances, the inventors have developed charge-transporting materials which, along with the ability to be used in various wet processes, provide thin films that, when used as a hole-injecting layer in an organic EL device, enable excellent EL device characteristics to be attained. The inventors have also developed compounds which have a good solubility in organic solvents used in such materials (see, for example, Patent Documents 1 to 4). CN 102 219 700 A discloses compound FPy, which comprises as substituents at the 9 position of fluorene, alkyl groups of 2 to 20 carbon atoms which include at least one ether structure.

JP2013234169 describes an aromatic amine derivative and an organic electroluminescence device using the same. The invention also relates to an electronic apparatus equipped with the organic EL device (Patent document 5).

### PRIOR ART DOCUMENTS

### PATENT DOCUMENTS

Patent Document 1: WO 2008/032616
Patent Document 2: WO 2008/129947
Patent Document 3: WO 2006/025342
Patent Document 4: WO 2010/058777
Patent Document 5: JP2013234169

### SUMMARY OF THE INVENTION

### PROBLEMS TO BE SOLVED BY THE INVENTION

It is therefore an object of the invention to provide a charge-transporting varnish which, as with the art disclosed to date in the foregoing patent publications, is suitable for use with various wet processes, and moreover gives a thin film that, when employed as the hole-injecting layer in an organic EL device, enables excellent device characteristics to be attained.

### MEANS FOR SOLVING THE PROBLEMS

The inventors have conducted extensive investigations in order to achieve the above object. As a result, they have discovered that a thin film obtained from a varnish which includes a charge-transporting substance consisting of a specific arylamine derivative, a dopant and an organic solvent has a high charge transportability, and that when such a thin film is employed as the hole-injecting layer in an organic EL device, it enables excellent device characteristics and an excellent durability to be attained.

Accordingly, the invention provides the following charge-transporting varnish, charge-transporting thin film and organic EL device.
1. A charge-transporting varnish containing a charge-transporting substance consisting of the arylamine derivative of formula (1) below, a dopant, and an organic solvent 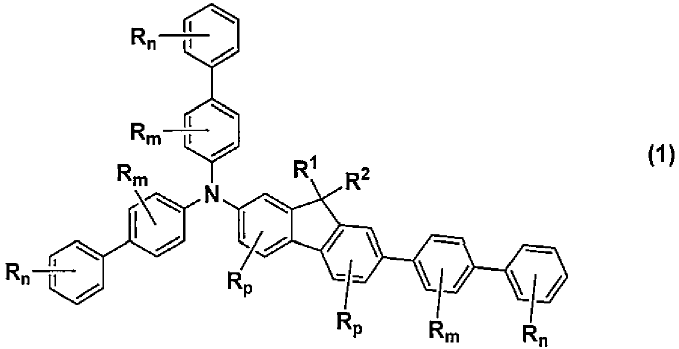
   wherein R¹ and R² are each independently an alkyl group of 2 to 20 carbon atoms which includes at least one ether structure, and some or all of the hydrogen atoms bonded to carbon atoms on these groups may be substituted with halogen atoms;
   each R is independently a halogen atom, a nitro group, a cyano group, an alkyl group of 1 to 20 carbon atoms which may be substituted with Z¹, an alkenyl group of 2 to 20 carbon atoms which may be substituted with Z¹, an alkynyl group of 2 to 20 carbon atoms which may be substituted with Z¹, an alkoxy group of 1 to 20 carbon atoms which may be substituted with Z¹, an alkenyloxy group of 2 to 20 carbon atoms which may be substituted with Z¹, or an alkynyloxy group of 2 to 20 carbon atoms which may be substituted with Z¹;
   Z¹ is a halogen atom, a nitro group, a cyano group, an aryl group of 6 to 20 carbon atoms which may be substituted with Z², a heteroaryl group of 2 to 20 carbon atoms which may be substituted with Z², an alkoxy group of 1 to 20 carbon atoms which may be substituted with Z², an alkenyloxy group of 2 to 20 carbon atoms which may be substituted with Z², an alkynyloxy group of 2 to 20 carbon atoms which may be substituted with Z², an aryl group of 6 to 20 carbon atoms which may be substituted with Z², or a heteroaryl group of 2 to 20 carbon atoms which may be substituted with Z²;
   Z² is a halogen atom, a nitro group or a cyano group; and
   each subscript n is independently an integer from 0 to 5, each subscript m is independently an integer from 0 to 4, and each subscript p is independently an integer from 0 to 3.
2. The charge-transporting varnish of 1 above, wherein the subscripts n, m and p are all 0.
3. The charge-transporting varnish of any of 1 or 2 above, wherein the dopant is a heteropolyacid.
4. A charge-transporting thin film comprising a charge-transporting substance consisting of formula (1) above and a dopant.
5. An organic electroluminescent device which includes the charge-transporting thin film of 4 above.
6. The organic electroluminescent device of 5 above, wherein the charge-transporting thin film is a hole-injecting layer.
7. An arylamine derivative of formula (1') 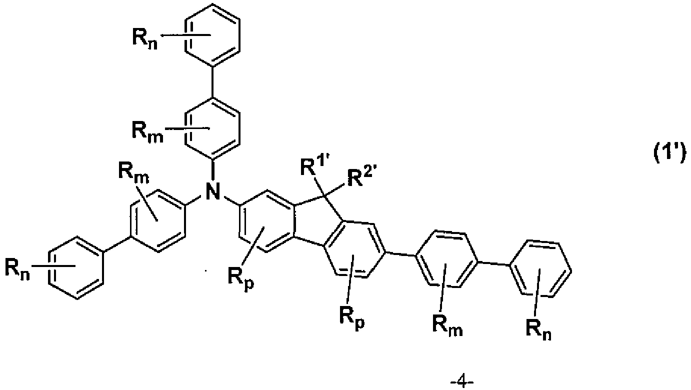
   wherein R^{1'} and R^{2'} are each independently an alkyl group of 2 to 20 carbon atoms which includes at least one ether structure, and some or all of the hydrogen atoms bonded to carbon atoms on these groups may be substituted with halogen atoms;
   each R is independently a halogen atom, a nitro group, a cyano group, an alkyl group of 1 to 20 carbon atoms which may be substituted with Z¹, an alkenyl group of 2 to 20 carbon atoms which may be substituted with Z¹ an alkynyl group of 2 to 20 carbon atoms which may be substituted with Z¹, an alkoxy group of 1 to 20 carbon atoms which may be substituted with Z¹, an alkenyloxy group of 2 to 20 carbon atoms which may be substituted with Z¹, or an alkynyloxy group of 2 to 20 carbon atoms which may be substituted with Z¹;
   Z¹ is a halogen atom, a nitro group, a cyano group, an aryl group of 6 to 20 carbon atoms which may be substituted with Z², a heteroaryl group of 2 to 20 carbon atoms which may be substituted with Z², an alkoxy group of 1 to 20 carbon atoms which may be substituted with Z², an alkenyloxy group of 2 to 20 carbon atoms which may be substituted with Z², an alkynyloxy group of 2 to 20 carbon atoms which may be substituted with Z², an aryl group of 6 to 20 carbon atoms which may be substituted with Z², or a heteroaryl group of 2 to 20 carbon atoms which may be substituted with Z²;
   Z² is a halogen atom, a nitro group or a cyano group; and
   each subscript n is independently an integer from 0 to 5, each subscript m is independently an integer from 0 to 4, and each subscript p is independently an integer from 0 to 3.

### ADVANTAGEOUS EFFECTS OF THE INVENTION

The arylamine derivative used in the present invention readily dissolves in organic solvents. By dissolving this together with a dopant in an organic solvent, a charge-transporting varnish can easily be prepared.

A thin film produced from the charge-transporting varnish of the invention exhibits high charge transportability, and therefore can be suitably used as thin films for organic EL devices and other electronic devices. In particular, by using this thin film as the hole-injecting layer for an organic EL device, organic EL devices having excellent characteristics can be obtained.

The charge-transporting varnish of the invention can reproducibly form thin films having excellent charge-transporting properties, even when various wet processes capable of film formation over a large area, such as spin coating and slip coating, are used, and therefore is capable of fully accommodating recent advances in the field of organic EL devices.

### DESCRIPTION OF THE EMBODIMENTS

### [Charge-Transporting Substance]

The charge-transporting varnish of the invention contains a charge-transporting substance consisting of an arylamine derivative of formula (1) below. In the invention, "charge transportability" is synonymous with electrical conductivity, and is also synonymous with hole transportability. The charge-transporting substance may be one that itself has charge transportability, or may be one that has charge transportability when used together with an electron-accepting substance. The charge-transporting varnish may be one that itself has charge transportability, or may be one where a solid film obtained therefrom has charge transportability.

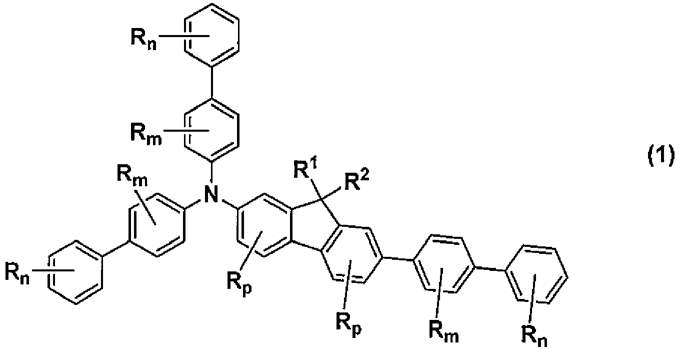

In the formula, R¹ and R² are each independently an alkyl group of 2 to 20 carbon atoms which includes at least one ether structure. Some or all of the hydrogen atoms bonded to carbon atoms in these groups may be substituted with halogen atoms.

Each R is independently a halogen atom, a nitro group, a cyano group, an alkyl group of 1 to 20 carbon atoms which may be substituted with Z¹ an alkenyl group of 2 to 20 carbon atoms which may be substituted with Z¹, an alkynyl group of 2 to 20 carbon atoms which may be substituted with Z¹ an alkoxy group of 1 to 20 carbon atoms which may be substituted with Z¹, an alkenyloxy group of 2 to 20 carbon atoms which may be substituted with Z¹, or an alkynyloxy group of 2 to 20 carbon atoms which may be substituted with Z¹.

Z¹ is a halogen atom, a nitro group, a cyano group, an aryl group of 6 to 20 carbon atoms which may be substituted with Z², a heteroaryl group of 2 to 20 carbon atoms which may be substituted with Z², an alkoxy group of 1 to 20 carbon atoms which may be substituted with Z², an alkenyloxy group of 2 to 20 carbon atoms which may be substituted with Z², an alkynyloxy group of 2 to 20 carbon atoms which may be substituted with Z², an aryl group of 6 to 20 carbon atoms which may be substituted with Z², or a heteroaryl group of 2 to 20 carbon atoms which may be substituted with Z².

Z² is a halogen atom, a nitro group or a cyano group.

The halogen atom is exemplified by fluorine, chlorine, bromine and iodine atoms.

The alkyl group of 1 to 20 carbon atoms may be linear, branched or cyclic. Examples include linear or branched alkyl groups of 1 to 20 carbon atoms, such as methyl, ethyl, n-propyl, isopropyl, n-butyl, isobutyl, s-butyl, t-butyl, n-pentyl, n-hexyl, n-heptyl, n-octyl, n-nonyl and n-decyl groups; and cyclic alkyl groups of 3 to 20 carbon atoms, such as cyclopropyl, cyclobutyl, cyclopentyl, cyclohexyl, cycloheptyl, cyclooctyl, cyclononyl, cyclodecyl, bicyclobutyl, bicyclopentyl, bicyclohexyl, bicycloheptyl, bicyclooctyl, bicyclononyl and bicyclodecyl groups.

The alkenyl group of 2 to 20 carbon atoms may be linear, branched or cyclic. Examples include ethenyl, n-1-propenyl, n-2-propenyl, 1-methylethenyl, n-1-butenyl, n-2-butenyl, n-3-butenyl, 2-methyl-1-propenyl, 2-methyl-2-propenyl, 1-ethylethenyl, 1-methyl-1-propenyl, 1-methyl-2-propenyl, n-1-pentenyl, n-1-decenyl and n-1-eicosenyl groups.

The alkynyl group of 2 to 20 carbon atoms may be linear, branched or cyclic. Examples include ethynyl, n-1-propynyl, n-2-propynyl, n-1-butynyl, n-2-butynyl, n-3-butynyl, 1-methyl-2-propynyl, n-1-pentynyl, n-2-pentynyl, n-3-pentynyl, n-4-pentynyl, 1-methyl-n-butynyl, 2-methyl-n-butynyl, 3-methyl-n-butynyl, 1,1-dimethyl-n-propynyl, n-1-hexynyl, n-1-decynyl, n-1-pentadecynyl and n-1-eicosynyl groups.

Examples of aryl groups of 6 to 20 carbon atoms include phenyl, 1-naphthyl, 2-naphthyl, 1-anthryl, 2-anthryl, 9-anthryl, 1-phenanthryl, 2-phenanthryl, 3-phenanthryl, 4-phenanthryl and 9-phenanthryl groups.

Examples of heteroaryl groups of 2 to 20 carbon atoms include 2-thienyl, 3-thienyl, 2-furanyl, 3-furanyl, 2-oxazolyl, 4-oxazolyl, 5-oxazolyl, 3-isooxazolyl, 4-isooxazolyl, 5-isooxazolyl, 2-thiazolyl, 4-thiazolyl, 5-thiazolyl, 3-isothiazolyl, 4-isothiazolyl, 5-isothiazolyl, 2-imidazolyl, 4-imidazolyl, 2-pyridyl, 3-pyridyl and 4-pyridyl.

The alkoxy group of 1 to 20 carbon atoms may be linear, branched or cyclic. Examples include linear or branched alkoxy groups of 1 to 20 carbon atoms, such as methoxy, ethoxy, n-propoxy, isopropoxy, n-butoxy, isobutoxy, s-butoxy, t-butoxy, n-pentyloxy, n-hexyloxy, n-heptyloxy, n-octyloxy, n-nonyloxy and n-decyloxy groups; and cyclic alkoxy groups of 3 to 20 carbon atoms, such as cyclopropyloxy, cyclobutyloxy, cyclopentyloxy, cyclohexyloxy, cycloheptyloxy, cyclooctyloxy, cyclononyloxy, cyclodecyloxy, bicyclobutyloxy, bicyclopentyloxy, bicyclohexyloxy, bicycloheptyloxy, bicyclooctyloxy, bicyclononyloxy and bicyclodecyloxy groups.

The alkenyloxy group of 2 to 20 carbon atoms may be linear, branched or cyclic. Examples include ethenyloxy, n-1-propenyloxy, n-2-propenyloxy, 1-methylethenyloxy, n-1-butenyloxy, n-2-butenyloxy, n-3-butenyloxy, 2-methyl-1-propenyloxy, 2-methyl-2-propenyloxy, 1-ethylethenyloxy, 1-methyl-1-propenyloxy, 1-methyl-2-propenyloxy, n-1-pentenyloxy, n-1-decenyloxy and n-1-eicosenyloxy groups.

The alkynyloxy group of 2 to 20 carbon atoms may be linear, branched or cyclic. Examples include ethynyloxy, n-1-propynyloxy, n-2-propynyloxy, n-1-butynyloxy, n-2-butynyloxy, n-3-butynyloxy, 1-methyl-2-propynyloxy, n-1-pentynyloxy, n-2-pentynyloxy, n-3-pentynyloxy, n-4-pentynyloxy, 1-methyl-n-butynyloxy, 2-methyl-n-butynyloxy, 3-methyl-n-butynyloxy, 1,1-dimethyl-n-propynyloxy, n-1-hexynyloxy, n-1-decynyloxy, n-1-pentadecynyloxy and n-1-eicosynyloxy groups.

The alkyl group of 2 to 20 carbon atoms which includes at least one ether structure is exemplified by linear or branched alkyl groups in which at least one methylene group is substituted with an oxygen atom. However, it is not one in which a methylene group bonded to a fluorene structure is substituted with an oxygen atom; nor is it one in which a neighboring methylene group is at the same time substituted with an oxygen atom. Taking into account the availability of the starting compounds, such a group is preferably a group of formula (A), and more preferably a group of formula (B).

-(R^{A}O)ᵣ-R^{B} (A)

-(CH₂CH₂O)ᵣ-CH₃ (B)

In the formulas, R^{A} is a linear or branched alkylene group of 1 to 4 carbon atoms, R^{B} is a linear or branched alkyl group of from 1 to [20 - (number of carbon atoms in R^{A}) × r] carbon atoms, and the subscript r is an integer from 1 to 9. From the standpoint of compatibility with the dopant, r is preferably at least 2, and more preferably at least 3; from the standpoint of the availability of the starting compounds, r is preferably 5 or less, and more preferably 4 or less.

Examples of alkyl groups of 2 to 20 carbon atoms which include at least one ether structure include -CH₂OCH₃, -CH₂OCH₂CH₃, -CH₂O(CH₂)₂CH₃, -CH₂OCH(CH₃)₂, -CH₂O(CH₂)₃CH₃, -CH₂OCH₂CH(CH₃)₂, -CH₂O(CH₃)₃, -CH₂O(CH₂)₄CH₃, -CH₂OCH(CH₃)(CH₂)₂CH₃, -CH₂O(CH₂)₂CH(CH₃)₂, -CH₂OCH(CH₃)(CH₂)₃CH₃, -CH₂O(CH₂)₅CH₃, -CH₂OCH₂CH(CH₃)(CH₂)₂CH₃, -CH₂O(CH₂)₂CH(CH₃)CH₂CH₃, -CH₂O(CH₂)₃CH(CH₃)₂, -CH₂OC(CH₃)₂(CH₂)₂CH₃, -CH₂OCH(CH₂CH₃)(CH₂)₂CH₃, -CH₂OC(CH₃)₂CH(CH₃)₂, -CH₂O(CH₂)₆CH₃, -CH₂O(CH₂)₇CH₃, -CH₂OCH₂CH(CH₂CH₃)(CH₂)₃CH₃, -CH₂O(CH₂)ₛCH₃, -CH₂O(CH₂)₉CH₃, -CH₂O(CH₂)₁₀CH₃, -CH₂O(CH₂)₁₁CH₃, -CH₂O(CH₂)₁₂CH₃, -CH₂O(CH₂)₁₃CH₃, -CH₂O(CH₂)₁₄CH₃, -CH₂O(CH₂)₁₅CH₃, -CH₂O(CH₂)₁₆CH₃, -CH₂O(CH₂)₁₇CH₃, -CH₂O(CH₂)₁₈CH₃, -CH₂CH₂OCH₃, -CH₂CH₂OCH₂CH₃, -CH₂CH₂O(CH₂)₂CH₃, -CH₂CH₂OCH(CH₃)₂, -CH₂CH₂O(CH₂)₃CH₃, -CH₂CH₂OCH₂CH(CH₃)₂, -CH₂CH₂OC(CH₃)₃, -CH₂CH₂O(CH₂)₄CH₃, -CH₂CH₂OCH(CH₃)(CH₂)₂CH₃, -CH₂CH₂OCH₂CH(CH₃)₂, -CH₂CH₂O(CH₂)₂CH(CH₃)₂, -CH₂CH₂OC(CH₃)₃, -CH₂CH₂OCH(CH₃)(CH₂)₃CH₃, -CH₂CH₂O(CH₂)₅CH₃, -CH₂CH₂OCH(CH₃)(CH₂)₃CH₃, -CH₂CH₂OCH₂CH(CH₃)(CH₂)₂CH₃, -CH₂CH₂O(CH₂)₂CH(CH₃)CH₂CH₃, -CH₂CH₂O(CH₂)₃CH(CH₃)₂, -CH₂CH₂OC(CH₃)₂(CH₂)₂CH₃, -CH₂CH₂OCH(CH₂CH₃)(CH₂)₂CH₃, -CH₂CH₂OC(CH₃)₂CH(CH₃)₂, -CH₂CH₂O(CH₂)₆CH₃, -CH₂CH₂O(CH₂)₇CH₃, -CH₂CH₂OCH₂CH(CH₂CH₃)(CH₂)₃CH₃, -CH₂CH₂O(CH₂)₈CH₃, -CH₂CH₂O(CH₂)₉CH₃, -CH₂CH₂O(CH₂)₁₀CH₃, -CH₂CH₂O(CH₂)₁₁CH₃, -CH₂CH₂O(CH₂)₁₂CH₃, -CH₂CH₂O(CH₂)₁₃CH₃, -CH₂CH₂O(CH₂)ₜ₄CH₃, -CH₂CH₂O(CH₂)₁₅CH₃, -CH₂CH₂O(CH₂)₁₆CH₃, -CH₂CH₂O(CH₂)₁₇CH₃, -CH₂CH₂CH₂OCH₃, -CH₂CH₂CH₂OCH₂CH₃, -CH₂CH₂CH₂O(CH₂)₃CH₃, -CH₂CH₂CH₂OCH(CH₃)₂, -CH₂CH₂CH₂O(CH₂)₃CH₃, -CH₂CH₂CH₂OCH₂CH(CH₃)₂, -CH₂CH₂CH₂OC(CH₃)₃, -CH₂CH₂CH₂O(CH₂)₄CH₃, -CH₂CH₂CH₂OCH(CH₃)(CH₂)₂CH₃, -CH₂CH₂CH₂OCH₂CH(CH₃)₂, -CH₂CH₂CH₂O(CH₂)₂CH(CH₃)₂) -CH₂CH₂CH₂OC(CH₃)₃, -CH₂CH₂CH₂OCH(CH₃)(CH₂)₃CH₃, -CH₂CH₂CH₂O(CH₂)₅CH₃, -CH₂CH₂CH₂OCH(CH₃)(CH₂)₃CH₃, -CH₂CH₂CH₂OCH₂CH(CH₃)(CH₂)₂CH₃, -CH₂CH₂CH₂O(CH₂)₂CH(CH₃)CH₂CH₃₎ -CH₂CH₂CH₂O(CH₂)₃CH(CH₃)₂, -CH₂CH₂CH₂OC(CH₁)₂(CH₂)₂CH₃, -CH₂CH₂CH₂OCH(CH₂CH₃)(CH₂)₂CH₃, -CH₂CH₂CH₂OC(CH₃)₂CH(CH₃)₂, -CH₂CH₂CH₂O(CH₂)₆CH₃, -CH₂CH₂CH₂O(CH₂)₇CH₃, -CH₂CH₂CH₂OCH₂CH(CH₂CH₃)(CH₂)₃CH₃, -CH₂CH₂OCH₂CH₂OCH₃₎ -CH₂CH₂OCH₂CH₂OCH₂CH₂OCH₃, -CH₂CH₂OCH₂CH₂OCH₂CH₂OCH₂CH₂OCH₃, -CH₂CH₂OCH₂CH₂OCH₂CH₂OCH₂CH₂OCH₂CH₂OCH₃, -CH₂CH₂OCH₂CH₂OCH₂CH₂OCH₂CH₂OCH₂CH₂OCH₂CH₂OCH₃, -CH₂CH₂OCH₂CH₂OCH₂CH₂OCH₂CH₂OCH₂CH₂OCH₂CH₂OCH₂CH₂OCH₃, -CH₂CH₂OCH₂CH₂OCH₂CH₂OCH₂CH₂OCH₂CH₂OCH₂CH₂OCH₂CH₂OCH₂CH₂OCH₃, -CH₂CH₂CH₂OCH₂CH₂CH₂OCH₃, -CH₂CH₂CH₂OCH₂CH₂CH₂OCH₂CH₂CH₂OCH₃, -CH₂CH₂CH₂OCH₂CH₂CH₂OCH₂CH₂CH₂OCH₂CH₂CH₂OCH₃, -CH₂CH₂CH₂OCH₂CH₂CH₂OCH₂CH₂CH₂OCH₂CH₂CH₂OCH₂CH₂CH₂OCH₃, -CH₂CH₂CH₂OCH₂CH₂CH₂OCH₂CH₂CH₂OCH₂CH₂CH₂OCH₂CH₂CH₂OCH₂CH₂CH₂OCH₃, -CH₂CH₂CH₂CH₂OCH₂CH₂CH₂CH₂OCH₂CH₂CH₂CH₂OCH₃, -CH₂CH₂CH₂CH₂OCH₂CH₂CH₂CH₂OCH₂CH₂CH₂CH₂OCH₂CH₂CH₂CH₂OCH₃, -CH₂CH₂OCH₂CH₂OCH₂CH₂OCH₂CH₂OCH₂CH₂OCH₂CH₂OCH₂CH₃, -CH₂CH₂OCH₂CH₂OCH₂CH₂OCH₂CH₂OCH₂CH₂OCH₂CH₂OCH₂CH₂OCH₂CH₃, -CH₂CH₂OCH₂CH₂OCH₂CH₂OCH₂CH₂OCH₂CH₂OCH₂CH₂OCH₂CH₂OCH₂CH₂OCH₂CH₃, -CH₂CH₂OCH₂CH₂OCH₂CH₃, -CH₂CH₂OCH₂CH₂OCH₂CH₂OCH₂CH₃, -CH₂CH₂CH₂O(CH₂)₈CH₃, -CH₂CH₂CH₂O(CH₂)₉CH₃, -CH₂CH₂CH₂O(CH₂)₁₀CH₃, -CH₂CH₂CH₂O(CH₂)₁₁CH₃, -CH₂CH₂CH₂O(CH₂)₁₂CH₃, -CH₂CH₂CH₂O(CH₂)₁₃CH₃, -CH₂CH₂CH₂O(CH₂)₁₄CH₃, -CH₂CH₂CH₂O(CH₂)₁₅CH₃ and -CH₂CH₂CH₂O(CH₂)₁₆CH₃.

R¹ and R² are an alkyl group having at least one ether structure as defined in claim 1.

In formula (1), each n is independently an integer from 0 to 5, each m is independently an integer from 0 to 4, and each p is independently an integer from 0 to 3. From the standpoint of enhancing the charge transportability of the arylamine derivative of the invention, n, m and p are, each independently, preferably from 0 to 2, more preferably 0 or 1, and most preferably 0. It is especially preferable for n, m and p to all be 0.

### [Method of Preparing Arylamine Derivative]

The arylamine derivative can be synthesized using a known coupling reaction after first synthesizing an intermediate of formula (1') in accordance with Scheme A below. 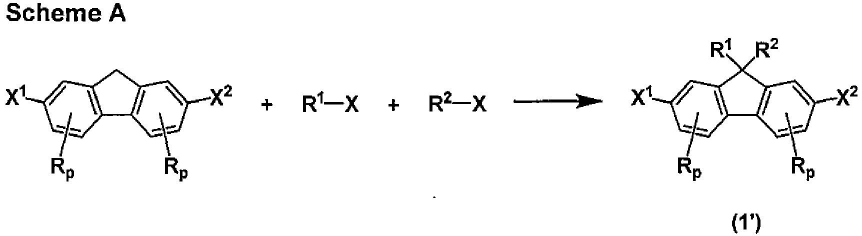 Here, R¹, R², R and p are as defined above; and X, X¹ and X² are each independently a halogen atom or a pseudo-halogen group.

The halogen atom is exemplified by fluorine, chlorine, bromine and iodine atoms. The pseudo-halogen group is exemplified by fluoroalkylsulfonyloxy groups such as methanesulfonyloxy, trifluoromethanesulfonyloxy and nanofluorobutanesulfonyloxy groups; and aromatic sulfonyloxy groups such as benzenesulfonyloxy and toluenesulfonyloxy groups.

Preferred examples of the solvent used in the Scheme A reaction include aliphatic hydrocarbons (e.g., pentane, n-hexane, n-octane, n-decane, decalin), halogenated aliphatic hydrocarbons (e.g., dichloromethane, 1,2-dichloroethane, carbon tetrachloride), aromatic hydrocarbons (e.g., benzene, toluene, o-xylene, m-xylene, p-xylene, mesitylene), ethers (e.g., diethyl ether, diisopropyl ether, t-butyl methyl ether, tetrahydrofuran, 1,4-dioxane, 1,2-dimethoxyethane, 1,2-diethoxyethane), amides (e.g., N,N-dimethylformamide, N,N-dimethylacetamide), ureas (e.g., N,N-dimethylimidazolidinone, tetramethylurea), sulfoxides (e.g., dimethylsulfoxide, sulfolane), nitriles (e.g., acetonitrile, propionitrile, butyronitrile), and water. The solvent is more preferably, for example, toluene, tetrahydrofuran, dimethylsulfoxide or water.

Examples of the catalyst used in the Scheme A reaction include sodium iodide, potassium iodide, cesium iodide, tetrabutylammonium fluoride, tetrabutylammonium chloride, tetrabutylammonium bromide, tetrabutylammonium iodide, benzyltriethylammonium chloride, benzyltriethylammonium bromide, benzyltriethylammonium iodide and methyltri-n-octylammonium chloride.

The reaction temperature is typically set as appropriate between the melting point and the boiling point of the solvent, and is generally from 0 to 140°C. The reaction time is generally from 0.1 to 100 hours.

Examples of methods for synthesizing the arylamine derivative from the intermediate of formula (1') by using a coupling reaction are shown below.

First, as shown in Scheme B below, an intermediate of formula (1") is synthesized by reacting an intermediate of formula (1') with a biphenylboric acid compound in the presence of a catalyst. 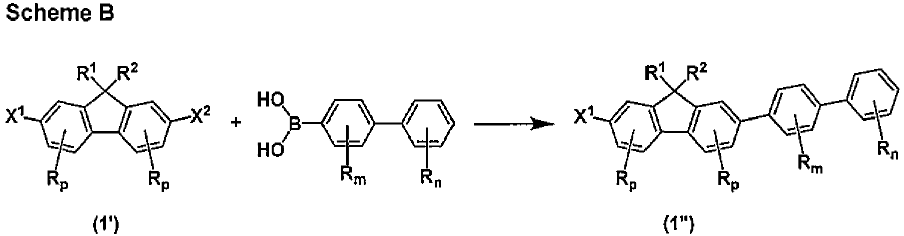 In the formulas, R¹, R², R, X¹, X², n, m and p are as defined above.

Next, as shown in Scheme C below, an arylamine derivative of formula (1) can be synthesized by a cross-coupling reaction between an intermediate of formula (1") and a bis(biphenylyl)amine compound in the presence of a catalyst. 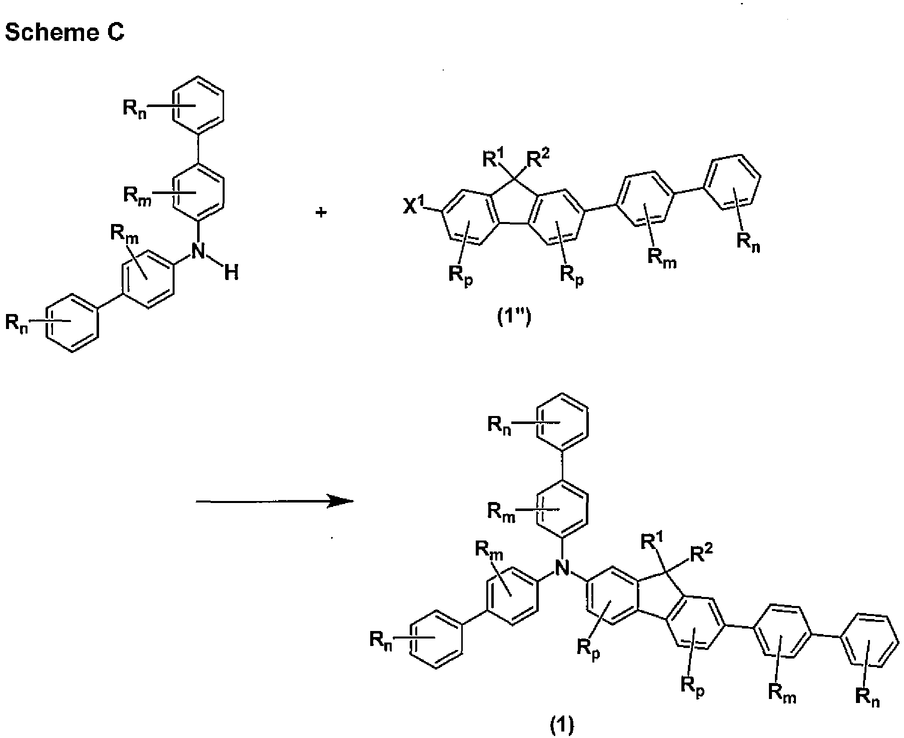 In the formulas, R¹, R², R, X¹, n, m and p are as defined above.

The catalyst used in the reactions in Schemes B and C is exemplified by palladium catalysts such as [1,1' -bis(diphenylphosphino)ferrocene] palladium(II) dichloride (PdCl₂(dppf)), tetrakis(triphenylphosphine)palladium (Pd(PPh₃)₄), bis(triphenylphosphine)dichloropalladium (Pd(PPh₃)₂Cl₂), bis(benzylideneacetone)palladium (Pd(dba)₂), tris(benzylideneacetone)dipalladium (Pd₂(dba)₃) and bis (tri-t-butylphosphine)palladium (Pd(P-t-Bu₃)₂).

Preferred examples of the solvent used in the reactions in Schemes B and C include aromatic hydrocarbons (e.g., benzene, toluene, o-xylene, m-xylene, p-xylene, mesitylene, cumene), ethers (e.g., diethyl ether, diisopropyl ether, t-butyl methyl ether, tetrahydrofuran, 1,4-dioxane, 1,2-dimethoxyethane, 1,2-diethoxyethane, diethylene glycol dimethyl ether), alcohols, e.g., methanol, ethanol, 1-propanol, 2-propanol), ketones (e.g., acetone, methyl ethyl ketone, methyl isobutyl ketone, di-n-butyl ketone, cyclohexanone), amides (e.g., N,N-dimethylformamide, N,N-dimethylacetamide), nitriles (acetonitrile, propionitrile, butyronitrile), and water.

The reaction temperature may be generally from the melting point of the solvent used or -50°C to the boiling point of the solvent, with the range of 0 to 140°C being preferred. The reaction time is generally from 0.1 to 100 hours.

Following reaction completion, the target arylamine derivative can be obtained by work-up in the usual manner.

### [Dopant]

The charge-transporting varnish of the invention contains a dopant. The dopant is not particularly limited; both inorganic dopants and organic dopants may be used.

Of these, in the most preferred embodiment, the charge-transporting varnish of the invention contains as the dopant a heteropolyacid. In this case, there can be obtained a thin film of excellent charge-transportability which exhibits not only a high ability to accept holes from transparent electrodes such as indium-tin oxide (ITO) and indium-zinc oxide (IZO), but also a high ability to accept holes from metal anodes such as aluminum.

"Heteropolyacid" refers to a polyacid having a structure in which a heteroatom is positioned at the center of the molecule--typically the Keggin-type chemical structure shown in formula (A1) or the Dawson-type chemical structure shown in formula (A2), and which is obtained by the condensation of an isopolyacid that is an oxoacid of vanadium (V), molybdenum (Mo), tungsten (W) or the like with an oxoacid of a different element. Examples of such oxoacids of a different element include primarily oxoacids of silicon (Si), phosphorus (P) and arsenic (As).

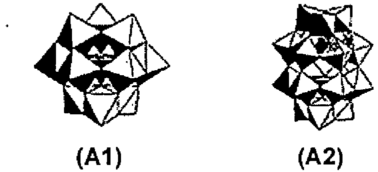

Examples of heteropolyacids include phosphomolybdic acid, silicomolybdic acid, phosphotungstic acid, silicotungstic acid and phosphotungstomolybdic acid. These may be used singly, or two or more may be used in combination. The heteropolyacid used in this invention may be acquired as a commercial product or may be synthesized by a known method.

In particular, when the dopant consists of a single heteropolyacid, this one heteropolyacid is preferably phosphotungstic acid or phosphomolybdic acid, and more preferably phosphotungstic acid. When the dopant consists of two or more heteropolyacids, at least one of these is preferably phosphotungstic acid or phosphomolybdic acid, and more preferably phosphotungstic acid.

Even a heteropolyacid having, in quantitative analysis such as elemental analysis, numbers for the elements which are higher or lower than in the structure indicated by the general formula may be used in this invention, provided it was acquired as a commercial product or was suitably synthesized according to a known method of synthesis.

For example, phosphotungstic acid is generally represented by the chemical formula H₃(PW₁₂O₄₀)·nH₂O and phosphomolybdic acid is generally represented by the chemical formula H₃(PMo₁₂O₄₀)·nH₂O. In quantitative analysis, regardless of whether the numbers for the elements P (phosphorus), O (oxygen) and W (tungsten) or Mo (molybdenum) within these formulas are high or low, so long as the heteropolyacid was acquired as a commercial product or suitably synthesized by a known method of synthesis, it may be used in this invention. In such cases, the mass of the heteropolyacid specified in this invention refers not to the mass of pure phosphotungstic acid within the product of synthesis or the commercial product (phosphotungstic acid content), but rather, in the form that is available as a commercial product or the form that can be isolated by a known method of synthesis, to the total mass in a state that includes water of hydration and other impurities.

The dopant is included within the charge-transporting varnish of the invention in a mass ratio relative to unity (1) for the charge-transporting substance of from about 0.05 to about 10.0. When a heteropolyacid alone is used as the dopant, the amount of the heteropolyacid may be set to a mass ratio relative to unity (1) for the charge-transporting substance of from about 0.05 to about 10.0, and preferably from about 0.1 to about 6.0.

### [Organic Solvent]

The organic solvent contained in the charge-transporting varnish of the invention is not particularly limited, provided it is one which dissolves well the arylamine derivative and the dopant. However, the organic solvent preferably includes an amide, and more preferably includes an acid amide derivative of formula (2) below. 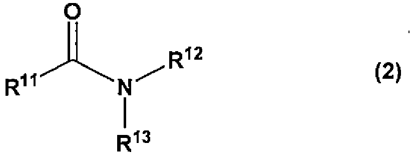 In the formula, R¹¹ represents a propyl or isopropyl group, preferably an isopropyl group. R¹² and R¹³ are each independently an alkyl group of 1 to 4 carbon atoms. The alkyl group of 1 to 4 carbon atoms may be linear, branched or cyclic. Examples include methyl, ethyl, n-propyl, isopropyl, cyclopropyl, n-butyl, isobutyl, s-butyl, t-butyl and cyclobutyl groups. Of these, R¹² and R¹³ are preferably methyl or ethyl groups.

Examples of the acid amide derivative of formula (2) include N,N-dimethylbutyramide, N,N-diethylbutyramide, N,N-methylethylbutyramide, N,N-dimethylisobutyramide (DMIB), N,N-diethylisobutyramide and N-ethyl-N-methylisobutyramide. Of these, DMIB is especially preferred.

The acid amide derivative of formula (2) may be synthesized by a substitution reaction between the corresponding carboxylic acid ester and amine, or a commercial product may be used.

Because the acid amide derivative of formula (2) is included, charge-transporting thin films of excellent flatness and uniformity can be more reproducibly obtained by various wet processes using the charge-transporting varnish of the invention than with conventional varnishes.

The organic solvent used in this invention may include organic solvents other than acid amide derivatives of formula (2). Illustrative examples of such other organic solvents include, but are not limited to, the following:
amides such as N-methylformamide, N,N-dimethylformamide, N,N-diethylformamide, N-methylacetamide, N,N-dimethylacetamide, N-methylpropionamide, 1,3-dimethyl-2-imidazolidinone and N-methylpyrrolidone;
glycols such as diethylene glycol, triethylene glycol, tetraethylene glycol, dipropylene glycol, 1,2-ethanediol (ethylene glycol), 1,2-propanediol (propylene glycol), 1,2-butanediol, 2,3-butanediol, 1,3-butanediol, 1,4-butanediol, 1,5-pentanediol, 2-methyl-2,4-pentanediol (hexylene glycol), 1,3-octylene glycol and 3,6-octylene glycol;
triols such as glycerol;
alkylene glycol monoalkyl ethers, including ethylene glycol monoalkyl ethers such as ethylene glycol monomethyl ether, ethylene glycol monoethyl ether, ethylene glycol monopropyl ether, ethylene glycol monoisopropyl ether, ethylene glycol monobutyl ether, ethylene glycol monoisobutyl ether and ethylene glycol monohexyl ether, and propylene glycol monoalkyl ethers such as propylene glycol monomethyl ether, propylene glycol monoethyl ether, propylene glycol monopropyl ether, propylene glycol monoisopropyl ether, propylene glycol monobutyl ether, propylene glycol monoisobutyl ether and propylene glycol monohexyl ether;
alkylene glycol monoaryl ethers, including ethylene glycol monoaryl ethers such as ethylene glycol monophenyl ether, and propylene glycol monoaryl ethers such as propylene glycol monophenyl ether;
alkylene glycol monoaralkyl ethers, including ethylene glycol monoaralkyl ethers such as ethylene glycol monobenzyl ether, and propylene glycol monoaralkyl ethers such as propylene glycol monobenzyl ether;
alkylene glycol alkoxyalkyl ethers, including ethylene glycol alkoxyalkyl ethers such as ethylene glycol butoxyethyl ether, and propylene glycol alkoxyalkyl ethers such as propylene glycol butoxyethyl ether;
alkylene glycol dialkyl ethers, including ethylene glycol dialkyl ethers such as ethylene glycol dimethyl ether, ethylene glycol diethyl ether, ethylene glycol dipropyl ether, ethylene glycol diisopropyl ether and ethylene glycol dibutyl ether, and propylene glycol dialkyl ethers such as propylene glycol dimethyl ether, propylene glycol diethyl ether, propylene glycol dipropyl ether, propylene glycol diisopropyl ether and propylene glycol dibutyl ether;
alkylene glycol monoalkyl ether acetates, including ethylene glycol monoalkyl ether acetates such as ethylene glycol monomethyl ether acetate, ethylene glycol monoethyl ether acetate, ethylene glycol monopropyl ether acetate, ethylene glycol monoisopropyl ether acetate and ethylene glycol monobutyl ether acetate, and propylene glycol monoalkyl ether acetates such as propylene glycol monomethyl ether acetate, propylene glycol monoethyl ether acetate, propylene glycol monopropyl ether acetate, propylene glycol monoisopropyl ether acetate and propylene glycol monobutyl ether acetate;
alkylene glycol monoacetates, including ethylene glycol monoacetates such as ethylene glycol monoacetate and propylene glycol monoacetates such as propylene glycol monoacetate;
alkylene glycol diacetates, including ethylene glycol diacetates such as ethylene glycol diacetate and propylene glycol diacetates such as propylene glycol diacetate;
dialkylene glycol monoalkyl ethers, including diethylene glycol monoalkyl ethers such as diethylene glycol monomethyl ether, diethylene glycol monoethyl ether, diethylene glycol monopropyl ether, diethylene glycol monoisopropyl ether, diethylene glycol monobutyl ether, diethylene glycol monoisobutyl ether and diethylene glycol monohexyl ether, and dipropylene glycol monoalkyl ethers such as dipropylene glycol monomethyl ether, dipropylene glycol monoethyl ether, dipropylene glycol monopropyl ether, dipropylene glycol monoisopropyl ether, dipropylene glycol monobutyl ether, dipropylene glycol monoisobutyl ether and dipropylene glycol monohexyl ether;
dialkylene glycol monoaryl ethers, including diethylene glycol monoaryl ethers such as diethylene glycol monophenyl ether, and dipropylene glycol monoaryl ethers such as dipropylene glycol monophenyl ether;
dialkylene glycol dialkyl ethers, including diethylene glycol dialkyl ethers such as diethylene glycol dimethyl ether, diethylene glycol diethyl ether, diethylene glycol dipropyl ether, diethylene glycol diisopropyl ether and diethylene glycol dibutyl ether, and dipropylene glycol dialkyl ethers such as dipropylene glycol dimethyl ether, dipropylene glycol diethyl ether, dipropylene glycol dipropyl ether, dipropylene glycol diisopropyl ether and dipropylene glycol dibutyl ether;
dialkylene glycol monoalkyl ether acetates, including diethylene glycol monoalkyl ether acetates such as diethylene glycol monomethyl ether acetate, diethylene glycol monoethyl ether acetate, diethylene glycol monopropyl ether acetate, diethylene glycol monoisopropyl ether acetate, diethylene glycol monobutyl ether acetate, diethylene glycol monoisobutyl ether acetate and diethylene glycol monohexyl ether acetate, and dipropylene glycol monoalkyl ether acetates such as dipropylene glycol monomethyl ether acetate, dipropylene glycol monoethyl ether acetate, dipropylene glycol monopropyl ether acetate, dipropylene glycol monoisopropyl ether acetate, dipropylene glycol monobutyl ether acetate, dipropylene glycol monoisobutyl ether acetate and dipropylene glycol monohexyl ether acetate;
trialkylene glycol monoalkyl ethers, including triethylene glycol monoalkyl ethers such as triethylene glycol monomethyl ether and triethylene glycol monoethyl ether, and tripropylene glycol monoalkyl ethers such as tripropylene glycol monomethyl ether and tripropylene glycol monoethyl ether;
trialkylene glycol dialkyl ethers, including triethylene glycol dialkyl ethers such as triethylene glycol dimethyl ether and triethylene glycol diethyl ether, and tripropylene glycol dialkyl ethers such as tripropylene glycol dimethyl ether and tripropylene glycol diethyl ether;
aliphatic alcohols, including linear aliphatic alcohols such as 1-propanol, 2-propanol, 1-butanol, 2-butanol, 1-pentanol, 1-hexanol, 1-heptanol, 1-nonanol, 1-decanol, 1-undecanol, 1-dodecanol and 1-tetradecanol, and cyclic aliphatic alcohols such as cyclohexanol and 2-methylcyclohexanol;
phenols such as phenol;
aromatic alcohols such as benzyl alcohol;
heterocycle-containing alcohols such as furfuryl alcohol;
hydrogenated heterocycle-containing alcohols such as tetrahydrofurfuryl alcohol;
dialkyl ethers such as diisopropyl ether, di-n-butyl ether and di-n-hexyl ether;
alkyl aryl ethers such as methyl phenyl ether, ethyl phenyl ether, n-butyl phenyl ether, benzyl (3-methylbutyl) ether, (2-methylphenyl) methyl ether, (3-methylphenyl) methyl ether and (4-methylphenyl) methyl ether;
alkyl aralkyl ethers such as ethyl benzyl ether;
cyclic alkyl monoethers such as 2-methylfuran, tetrahydrofuran and tetrahydropyran;
cyclic alkyl diethers such as 1,4-dioxane;
cyclic alkyl triethers such as trioxane;
diepoxyalkyl ethers such as diglycidyl ether;
alkyl esters exemplified by alkyl acetates, including linear or branched alkyl acetates such as ethyl acetate, n-propyl acetate, isopropyl acetate, n-butyl acetate, isobutyl acetate, s-butyl acetate, t-butyl acetate, n-pentyl acetate, (3-methylbutyl) acetate, n-hexyl acetate, (2-ethylbutyl) acetate and (2-ethylhexyl) acetate, and cyclic alkyl acetates such as cyclohexyl acetate and 2-methylcyclohexyl acetate; alkyl propionates, including linear or branched alkyl propionates such as ethyl propionate, n-propyl propionate, isopropyl propionate, n-butyl propionate, isobutyl propionate, s-butyl propionate, t-butyl propionate, n-pentyl propionate, (3-methylbutyl) propionate, n-hexyl propionate, (2-ethylbutyl) propionate and (2-ethylhexyl) propionate, and cyclic alkyl propionates such as cyclohexyl propionate and 2-methylcyclohexyl propionate; alkyl butyrates, including linear or branched alkyl butyrates such as ethyl butyrate, n-propyl butyrate, isopropyl butyrate, n-butyl butyrate, isobutyl butyrate, s-butyl butyrate, t-butyl butyrate, n-pentyl butyrate, (3-methylbutyl) butyrate, n-hexyl butyrate, (2-ethylbutyl) butyrate, (2-ethylhexyl) butyrate, and cyclic alkyl butyrates such as cyclohexyl butyrate and 2-methylcyclohexyl butyrate; and alkyl lactates, including linear or branched alkyl lactates such as ethyl lactate, n-propyl lactate, isopropyl lactate, n-butyl lactate, isobutyl lactate, s-butyl lactate, t-butyl lactate, n-pentyl lactate, (3-methylbutyl) lactate, n-hexyl lactate, (2-ethylbutyl) lactate and (2-ethylhexyl) lactate, and cyclic alkyl lactates such as cyclohexyl lactate and 2-methylcyclohexyl lactate;
aralkyl alkyl esters, including aralkyl acetates such as benzyl acetate, aralkyl propionates such as benzyl propionate, aralkyl butyrates such as benzyl butyrate, and aralkyl lactates such as benzyl lactate;
dialkyl ketones such as diethyl ketone, diisobutyl ketone, methyl ethyl ketone, methyl n-propyl ketone, methyl n-butyl ketone, methyl isobutyl ketone, methyl n-propyl ketone, methyl n-hexyl ketone, ethyl n-butyl ketone and di-n-propyl ketone;
cyclic alkenyl ketones such as isophorone;
cyclic alkyl ketones such as cyclohexanone;
hydroxydialkyl ketones such as 4-hydroxy-4-methyl-2-pentanone (diacetone alcohol);
heterocycle-containing aldehydes such as furfural;
linear or branched alkanes such as heptane, octane, 2,2,3-trimethylhexane, decane and dodecane;
alkylbenzenes such as toluene, xylene, o-xylene, m-xylene, p-xylene, mesitylene, tetralin and cyclohexylbenzene; and
cyclic alkanes such as cyclohexane, methylcyclohexane and ethylcyclohexane. These solvents may be of one type used alone, or two or more may be used in admixture.

When the charge-transporting varnish of the invention contains another organic solvent aside from the acid amide of formula (2), examples of this other organic solvent preferably include glycols, triols, alkylene glycol monoalkyl ethers, alkylene glycol dialkyl ethers, dialkylene glycol monoalkyl ethers and dialkylene glycol dialkyl ethers; more preferably include glycols, alkylene glycol monoalkyl ethers and dialkylene glycol monoalkyl ethers; even more preferably include diethylene glycol, triethylene glycol, dipropylene glycol, 1,2-ethanediol, 1,2-propanediol, 1,2-butanediol, 2,3-butanediol, 1,3-butanediol, 1,4-butanediol, ethylene glycol monomethyl ether, ethylene glycol monoethyl ether, ethylene glycol monopropyl ether, ethylene glycol monoisopropyl ether, ethylene glycol monobutyl ether, ethylene glycol monoisobutyl ether, propylene glycol monomethyl ether, propylene glycol monoethyl ether, propylene glycol monopropyl ether, propylene glycol monoisopropyl ether, propylene glycol monobutyl ether, propylene glycol monoisobutyl ether, diethylene glycol monomethyl ether, diethylene glycol monoethyl ether, diethylene glycol monopropyl ether, diethylene glycol monoisopropyl ether, diethylene glycol monobutyl ether, diethylene glycol monoisobutyl ether, dipropylene glycol monomethyl ether, dipropylene glycol monoethyl ether, dipropylene glycol monopropyl ether, dipropylene glycol monoisopropyl ether, dipropylene glycol monobutyl ether and dipropylene glycol monoisobutyl ether; and still more preferably include diethylene glycol, triethylene glycol, dipropylene glycol, 1,2-ethanediol, 1,2-propanediol, 1,2-butanediol, 2,3-butanediol, 1,3-butanediol, 1,4-butanediol, ethylene glycol monomethyl ether, propylene glycol monomethyl ether, diethylene glycol monomethyl ether and dipropylene glycol monomethyl ether.

When using the acid amide derivative of formula (2) and the other organic solvent together as the organic solvent, from the standpoint of reproducibly achieving a good film flatness and uniformity, the content of the acid amide derivative in the overall organic solvent is preferably at least 35 wt%, more preferably at least 50 wt%, even more preferably at least 55 wt%, still more preferably at least 60 wt%, and still yet more preferably at least 70 wt%, with the content being even more preferably, in order of increasing desirability, at least 75 wt%, or at least 80 wt%.

### [Charge-Transporting Varnish]

The charge-transporting varnish of the invention contains a charge-transporting substance consisting of the above-described arylamine derivative, a dopant and an organic solvent.

The varnish of the invention has a viscosity which is suitably set according to the thickness and other properties of the thin film to be produced and the solids concentration of the varnish, but is generally from 1 to 50 mPa·s at 25°C.

The solids concentration of the charge-transporting varnish of the invention is suitably set while taking into account such factors as the viscosity and surface tension of the varnish and the thickness of the thin film to be produced, but is generally about 0.1 to 10.0 wt%. To improve the ease of applying the varnish, the solids concentration is preferably about 0.5 to 5.0 wt%, and more preferably about 1.0 to 3.0 wt%.

The method of preparing the charge-transporting varnish is exemplified by, but not particularly limited to, a method in which the arylamine derivative is first dissolved in the solvent, and the dopant is added to the resulting solution; and a method in which a mixture of the arylamine derivative and the dopant is dissolved in the solvent. In cases where there are a plurality of organic solvents, the arylamine derivative and the dopant may first be dissolved in a solvent that dissolves them well, after which the other solvents may be added thereto; alternatively, the arylamine derivative and the dopant may be dissolved one after the other or at the same time in a mixed solvent formed of the plurality of organic solvents.

In this invention, from the standpoint of reproducibly obtaining thin films of high flatness, after the charge-transporting substance, dopant and the like have been dissolved in the organic solvent, it is desirable for the resulting charge-transporting varnish to be filtered using, for example, a submicron-order filter.

### [Charge-Transporting Thin Film]

The charge-transporting varnish of the invention is preferably a coating-type charge-transporting thin film-forming varnish. A charge-transporting thin film can be formed on a substrate by coating or otherwise applying this varnish onto a substrate and baking the applied varnish.

Examples of methods for applying the varnish include, but are not limited to, dipping, spin coating, transfer printing, roll coating, brush coating, ink-jet printing, spraying and slit coating. To reproducibly obtain a charge-transporting thin film having a high flatness, preferred methods of application include spin coating, inkjet coating and spraying. It is preferable to adjust the viscosity and surface tension of the varnish according to the method of application.

When using the varnish of the invention, in order to obtain a thin film having a uniform film surface and a high charge transportability, the baking atmosphere (such as an open-air atmosphere, an atmosphere of inert gas such as nitrogen, or a vacuum) must be selected while taking into account, for example, the types of dopants and solvents that are included together with the arylamine derivative contained in the invention. However, in most cases, a thin film which is uniform and has excellent charge transportability can be obtained by baking in an open-air atmosphere.

The baking temperature is suitably set in the range of about 100 to 260°C while taking into account such factors as the intended use of the resulting thin film and the degree of charge transportability to be imparted to the thin film. When the thin film thus obtained is to be used as a hole-injecting layer in an organic EL device, the baking temperature is preferably about 140 to 250°C, and more preferably about 145 to 240°C.

During baking, a temperature change in two or more steps may be applied for such purposes as to achieve more uniform film formability or to induce the reaction to proceed on the substrate. Heating may be carried out using a suitable apparatus such as a hot plate or an oven.

The thickness of the charge-transporting thin film is not particularly limited. However, when the thin film is to be used as a hole-injecting layer in an organic EL device, a film thickness of from 5 to 200 nm is preferred. Methods for changing the film thickness include, for example, changing the solids concentration in the varnish and changing the amount of solution on the substrate during application.

The charge-transporting thin film of the invention can be suitably used as a hole-injecting layer in an organic EL device, although use as a charge-transporting functional layer such as a hole-injecting-and-transporting layer is also possible.

### [Organic EL Device]

The organic EL device of the invention has a pair of electrodes and also has, between these electrodes, the above-described charge-transporting thin film of the invention.

Typical organic EL device configurations include, but are not limited to, those of (a) to (f) below. In these configurations, where necessary, an electron-blocking layer or the like may be provided between the light-emitting layer and the anode, and a hole-blocking layer or the like may be provided between the light-emitting layer and the cathode. Alternatively, the hole-injecting layer, hole-transporting layer or hole-injecting-and-transporting layer may also have the function of, for example, an electron-blocking layer; and the electron-injecting layer, electron-transporting layer or electron-injecting-and-transporting layer may also have the function of, for example, a hole-blocking layer.
(a) anode/hole-injecting layer/hole-transporting layer/light-emitting layer/electron-transporting layer/electron-injecting layer/cathode
(b) anode/hole-injecting layer/hole-transporting layer/light-emitting layer/electron-injecting-and-transporting layer/cathode
(c) anode/hole-injecting-and-transporting layer/light-emitting layer/electron-transporting layer/electron-injecting layer/cathode
(d) anode/hole-injecting-and-transporting layer/light-emitting layer/electron-injecting-and-transporting layer/cathode
(e) anode/hole-injecting layer/hole-transporting layer/light-emitting layer/cathode
(f) anode/hole-injecting-and-transporting layer/light-emitting layer/cathode

As used herein, "hole-injecting layer," "hole-transporting layer" and "hole-injecting-and-transporting layer" refer to layers which are formed between the light-emitting layer and the anode and which have the function of transporting holes from the anode to the light-emitting layer. When only one layer of hole-transporting material is provided between the light-emitting layer and the anode, this is a "hole-injecting-and-transporting layer"; when two or more layers of hole-transporting material are provided between the light-emitting layer and the anode, the layer that is closer to the anode is a "hole-injecting layer" and the other layer is a "hole-transporting layer." In particular, thin films having not only an ability to receive holes from the anode but also an excellent ability to inject holes into the hole-transporting layer or the light-emitting layer may be used as, respectively, a hole-injecting layer or a hole-injecting-and-transporting layer.

In addition, "electron-injecting layer," "electron-transporting layer" and "electron-injecting-and-transporting layer" refer to layers which are formed between the light-emitting layer and the cathode and which have the function of transporting electrons from the cathode to the light-emitting layer. When only one layer of electron-transporting material is provided between the light-emitting layer and the cathode, this is an "electron-injecting-and-transporting layer"; when two or more layers of electron-transporting material are provided between the light-emitting layer and the cathode, the layer that is closer to the cathode is an "electron-injecting layer" and the other layer is an "electron-transporting layer."

The "light-emitting layer" is an organic layer having a light-emitting function. When a doping system is used, this layer includes a host material and a dopant material. The function of the host material is primarily to promote the recombination of electrons and holes and to confine the resulting excitons within the emissive layer. The function of the dopant material is to cause the excitons obtained by recombination to efficiently luminesce. In the case of a phosphorescent device, the host material functions primarily to confine within the light-emitting layer the excitons generated by the dopant.

The materials and method employed to fabricate an organic EL device using the charge-transporting varnish of the invention are exemplified by, but not limited to, those described below.

The electrode substrate to be used is preferably cleaned beforehand by liquid washing with, for example, a cleaning agent, alcohol or pure water. When the substrate is an anode substrate, it is preferably subjected to surface treatment such as UV/ozone treatment or oxygen-plasma treatment just prior to use. However, surface treatment need not be carried out if the anode material is composed primarily of organic substances.

An example of a method of fabricating the organic EL device of the invention in which a thin-film obtained from the charge-transporting varnish of the invention serves as a hole-injecting layer is described below.

In this method, a hole-injecting layer is formed on an electrode by applying the charge-transporting varnish of the invention onto an anode substrate, and baking the applied varnish. A hole-transporting layer, light-emitting layer, electron-transporting layer, electron-injecting layer and cathode are then provided in this order on the hole-injecting layer. The hole-transporting layer, light-emitting layer, electron-transporting layer and electron-injecting layer may be formed by either a vapor deposition process or a coating process (wet process), depending on the properties of the material to be used.

Illustrative examples of anode materials include transparent electrodes such as indium-tin oxide (ITO) and indium-zinc oxide (IZO), and metal anodes made of a metal such as aluminum or an alloy of such a metal. An anode material on which planarizing treatment has been carried out is preferred. Use can also be made of polythiophene derivatives and polyaniline derivatives having high charge transportability.

Examples of other metals making up the metal anode include, but are not limited to, scandium, titanium, vanadium, chromium, manganese, iron, cobalt, nickel, copper, zinc, gallium, yttrium, zirconium, niobium, molybdenum, ruthenium, rhodium, palladium, cadmium, indium, scandium, lanthanum, cerium, praseodymium, neodymium, promethium, samarium, europium, gadolinium, terbium, dysprosium, holmium, erbium, thulium, ytterbium, hafnium, thallium, tungsten, rhenium, osmium, iridium, platinum, gold, titanium, lead, bismuth, and alloys thereof.

Specific examples of hole-transporting layer-forming materials include the following hole-transporting low-molecular-weight materials: triarylamines such as (triphenylamine) dimer derivatives, [(triphenylamine) dimer] spirodimer, N,N'-bis(naphthalen-l-yl)-N,N'-bis(phenyl)benzidine (α-NPD), N,N' -bis(naphthalen-2-yl)-N,N' -bis(phenyl)benzidine, N,N'-bis(3-methylphenyl)-N,N' -bis(phenyl)benzidine, N,N'-bis(3-methylphenyl)-N,N' -bis(phenyl)-9,9-spirobifluorene, N,N'-bis(naphthalen-1-yl)- N,N'-bis(phenyl)-9,9-spirobifluorene, N,N'-bis(3-methylphenyl)-N,N'-bis(phenyl)-9,9-dimethylfluorene, N,N'-bis(naphthalen-1-yl)-N,N'-bis(phenyl)-9,9-dimethylfluorene, N,N' -bis(3-methylphenyl)-N,N'-bis(phenyl)-9,9-diphenylfluorene, N,N'-bis(naphthalen-1-yl)-N,N'-bis(phenyl)-9,9-diphenylfluorene, N,N'-bis(naphthalen-1-yl)-N,N'-bis(phenyl)-2,2'-dimethylbenzidine, 2,2',7,7'-tetrakis(N,N-diphenylamino)-9,9-spirobifluorene, 9,9-bis[4-(N,N-bis-biphenyl-4-yl-amino)phenyl]-9H-fluorene, 9,9-bis[4-(N,N-bis-naphthalen-2-yl-amino)phenyl]-9H-fluorene, 9,9-bis[4-(N-naphthalen-1-yl-N-phenylamino)phenyl]-9H-fluorene, 2,2',7,7'-tetrakis[N-naphthalenyl(phenyl)amino]-9,9-spirobifluorene, N,N'-bis(phenanthren-9-yl)-N,N'-bis(phenyl)benzidine, 2,2'-bis[N,N-bis(biphenyl-4-yl)amino]-9,9-spirobifluorene, 2,2'-bis(N,N-diphenylamino)-9,9-spirobifluorene, di[4-(N,N-di(p-tolyl)amino)phenyl]cyclohexane, 2,2',7,7'-tetra(N,N-di(p-tolyl))amino-9,9-spirobifluorene, N,N,N',N'-tetra-naphthalen-2-yl-benzidine, N,N,N',N'-tetra(3-methylphenyl)-3,3'-dimethylbenzidine, N,N'-di(naphthalenyl)-N,N'-di(naphthalen-2-yl)benzidine, N,N,N',N'-tetra(naphthalenyl)benzidine, N,N'-di(naphthalen-2-yl)-N,N'-diphenylbenzidine-1-4-diamine, N¹,N⁴-diphenyl-N¹,N⁴-di(m-tolyl)benzene-1,4-diamine, N²,N²,N⁶,N⁶-tetraphenylnaphthalene-2,6-diamine, tris(4-(quinolin-8-yl)phenyl)amine, 2,2'-bis(3-(N,N-di(p-tolyl)amino)phenyl)biphenyl, 4,4',4"-tris[3-methylphenyl(phenyl)amino]triphenylamine (m-MTDATA) and 4,4',4"-tris[1-naphthyl(phenyl)amino]triphenylamine (1-TNATA); and oligothiophenes such as 5,5"-bis-{4-[bis(4-methylphenyl)amino]phenyl}-2,2':5',2"-terthiophene (BMA-3T).

Specific examples of light-emitting layer-forming materials include tris(8-quinolinolate) aluminum(III) (Alq₃), bis(8-quinolinolate) zinc(II) (Znq₂), bis(2-methyl-8-quinolinolate)-4-(p-phenylphenolate) aluminum(III) (BAlq), 4,4'-bis(2,2-diphenylvinyl)biphenyl,9,10-di(naphthalen-2-yl)anthracene, 2-t-butyl-9,10-di(naphthalen-2-yl)anthracene, 2,7-bis[9,9-di(4-methylphenyl)-fluoren-2-yl]-9,9-di(4-methylphenyl)fluorene, 2-methy 1-9,10-bis(naphthalen-2-yl)anthracene, 2-(9,9-spirobifluoren-2-yl)-9,9-spirobifluorene, 2,7-bis(9,9-spirobifluoren-2-yl)-9,9-spirobifluorene, 2-[9,9-di(4-methylphenyl)-fluoren-2-yl]-9,9-di(4-methylphenyl)fluorene, 2,2'-dipyrenyl-9,9-spirobifluorene,1,3,5-tris(pyren-1-yl)benzene, 9,9-bis[4-(pyrenyl)phenyl]-9H-fluorene, 2,2'-bi(9,10-diphenylanthracene), 2,7-dipyrenyl-9,9-spirobifluorene, 1,4-di(pyren-1-yl)benzene, 1,3-di(pyren-1-yl)benzene, 6,13-di(biphenyl-4-yl)pentacene, 3,9-di(naphthalen-2-yl)perylene, 3,10-di(naphthalen-2-y1)perylene, tris[4-(pyrenyl)phenyl]amine, 10,10'-di(biphenyl-4-yl)-9,9'-bianthracene, N,N'-di(naphthalen-1-yl)-N,N'-diphenyl[1,1':4',l":4",1"'-quaterphenyl]-4,4"'-diamine, 4,4'-di[10-(naphthalen-1-yl)anthracen-9-yl]biphenyl, dibenzo {[f,f']-4,4',7,7'-tetraphenyl}diindeno[1,2,3-cd:1',2',3'-lm]perylene, 1-(7-(9,9'-bianthracen-10-yl)-9,9-dimethyl-9H-fluoren-2-yl)pyrene, 1-(7-(9,9'-bianthracen-10-yl)-9,9-dihexyl-9H-fluoren-2-yl)pyrene, 1,3-bis(carbazol-9-yl)benzene, 1,3,5-tris(carbazol-9-yl)benzene, 4,4',4"-tris(carbazol-9-yl)triphenylamine, 4,4'-bis(carbazol-9-yl)biphenyl (CBP), 4,4'-bis(carbazol-9-yl)-2,2'-dimethylbiphenyl, 2,7-bis(carbazol-9-yl)-9,9-dimethylfluorene, 2,2',7,7'-tetrakis(carbazol-9-yl)-9,9-spirobifluorene, 2,7-bis(carbazol-9-yl)-9,9-di(p-tolyl)fluorene, 9,9-bis[4-(carbazol-9-yl)phenyl]fluorene, 2,7-bis(carbazol-9-yl)-9,9-spirobifluorene, 1,4-bis(triphenylsilyl)benzene, 1,3-bis(triphenylsilyl)benzene, bis(4-N,N-diethylamino-2-methylphenyl)-4-methylphenylmethane, 2,7-bis(carbazol-9-yl)-9,9-dioctylfluorene, 4,4"-di(triphenylsilyl)-p-terphenyl, 4,4'-di(triphenylsilyl)biphenyl, 9-(4-t-butylphenyl)-3,6-bis(triphenylsilyl)-9H-carbazole, 9-(4-t-butylphenyl)-3,6-ditrityl-9H-carbazole, 9-(4-t-butylphenyl)-3,6-bis(9-(4-methoxyphenyl)-9H-fluoren-9-yl)-9H-carbazole, 2,6-bis(3-(9H-carbazol-9-yl)phenyl)pyridine, triphenyl(4-(9-phenyl-9H-fluoren-9-yl)phenyl)silane, 9,9-dimethyl-N,N-diphenyl-7-(4-(1-phenyl-1H-benzo[d]imidazol-2-yl)phenyl-9H-fluoren-2-amine, 3,5-bis(3-(9H-carbazol-9-yl)phenyl)pyridine, 9,9-spirobifluoren-2-yl-diphenylphosphine oxide, 9,9'-(5-triphenylsilyl)-1,3-phenylene)bis(9H-carbazole), 3-(2,7-bis(diphenylphosphoryl)-9-phenyl-9H-fluoren-9-yl)-9-phenyl-9H-carbazole, 4,4,8,8,12,12-hexa(p-tolyl)-4H-8H-12H-12C-azadibenzo[cd,mn]pyrene, 4,7-di(9H-carbazol-9-yl)-1,10-phenanthroline, 2,2'-bis(4-(carbazol-9-yl)phenyl)biphenyl, 2,8-bis(diphenylphosphoryl)dibenzo[b,d]thiophene, bis(2-methylphenyl)diphenylsilane, bis[3,5-di(9H-carbazol-9-yl)phenyl]diphenylsilane, 3,6-bis(carbazol-9-yl)-9-(2-ethyl-hexyl)-9H-carbazole, 3-(diphenylphosphoryl)-9-(4-(diphenylphosphoryl)phenyl)-9H-carbazole and 3,6-bis[(3,5-diphenyl)phenyl]-9-phenylcarbazole. A light-emitting layer may be formed by co-vapor deposition of these materials with a light-emitting dopant.

Specific examples of light-emitting dopants include 3-(2-benzothiazolyl)-7-(diethylamino)coumarin, 2,3,6,7-tetrahydro-1,1,7,7-tetramethyl-1H,5H,11H-10-(2-benzothiazolyl)quinolidino-[9,9a,1gh]coumarin, quinacridone, N,N'-dimethylquinacridone, tris(2-phenylpyridine) iridium(III) (Ir(ppy)₃), bis(2-phenylpyridine)(acetylacetonate) iridium(III) (Ir(ppy)₂(acac)), tris[2-(p-tolyl)pyridine] iridium(III) (Ir(mppy)₃), 9,10-bis[N,N-di(p-tolyl)amino]anthracene, 9,10-bis [phenyl(m-tolyl)amino] anthracene, bis [2-(2-hydroxyphenyl)benzothiazolate] zinc(II), N¹⁰,N¹⁰,N¹⁰,N¹⁰-tetra(p-tolyl)-9,9'-bianthracene-10,10'-diamine, N¹⁰,N¹⁰,N¹⁰,N¹⁰-tetraphenyl-9,9'-bianthracene-10,10'-diamine, N¹⁰,N¹⁰-diphenyl-N¹⁰,N¹⁰-dinaphthalenyl-9,9'-bianthracene-10,10'-diamine, 4,4'-bis(9-ethyl-3-carbazovinylene)-1,1'-biphenyl, perylene, 2,5,8,11-tetra-t-butylperylene, 1,4-bis[2-(3-N-ethylcarbazolyl)vinyl]benzene, 4,4'-bis[4-(di-p-tolylamino)styryl]biphenyl, 4-(di-p-tolylamino)-4'-[(di-p-tolylamino)styryl]stilbene, bis[3,5-difluoro-2-(2-pyridyl)phenyl-(2-carboxypyridyl)] iridium(III), 4,4' -bis [4-(diphenylamino)styryl]biphenyl, bis(2,4-diffuorophenylpyridinato)tetrakis(1-pyrazolyl)borate iridium(III, N,N'-bis(naphthalen-2-yl)-N,N'-bis(phenyl)tris(9,9-dimethylfluorenylene), 2,7-bis {2-[phenyl(m-tolyl)amino]-9,9-dimethylfluoren-7-yl} -9,9-dimethylfluorene, N-(4-((E)-2-(6((E)-4-(diphenylamino)styryl)naphthalen-2-yl)vinyl)phenyl)-N-phenyl-benzenamine, fac-iridium(III) tris(1-phenyl-3-methylbenzimidazolin-2-ylidene-C,C²), mer-iridium(III) tris(1-phenyl-3-methylbenzimidazolin-2-ylidene-C,C²), 2,7-bis[4-(diphenylamino)styryl]-9,9-spirobifluorene, 6-methyl-2-(4-(9-(4-(6-methylbenzo[d]thiazol-2-yl)phenyl)anthracen-10-yl)phenyl)-benzo[d]thiazole, 1,4-di[4-(N,N-diphenyl)amino]styrylbenzene, 1,4-bis(4-(9H-carb azol-9-yl)styryl)benzene, (E)-6-(4-(diphenylamino)styryl)-N,N-diphenylnaphthalen-2-amine, bis(2,4-difluorophenylpyridinato)(5-(pyridin-2-yl)-1H-tetrazolate) iridium(III), bis(3-trifluoromethyl-5-(2-pyridyl)pyrazole)((2,4-difluorobenzyl)diphenylphosphinate) iridium(III), bis(3-trifluoromethyl-5-(2-pyridyl)pyrazolate)(benzyldiphenylphosphinate)iridium(III), bis(1-(2,4-difluorobenzyl)-3-methylbenzimidazolium)(3-(trifluoromethyl)-5-(2-pyridyl)-1,2,4-triazolate) iridium(III), bis(3-trifluoromethyl-5-(2-pyridyl)pyrazolate)(4',6'-difluorophenylpyridinate) iridium(III), bis(4',6'-difluorophenylpyridinato)(3,5-bis(trifluoromethyl)-2-(2'-pyridyl)pyrrolate) iridium(III), bis(4',6'-difluorophenylpyridinato)(3-(trifluoromethyl)-5-(2-pyridyl)-1,2,4-triazolate) iridium (III), (Z)-6-mesityl-N-(6-mesitylquinolin-2(1H)-ylidene)quinoline-2-amine-BF₂, (E)-2-(2-(4-(dimethylamino)styryl)-6-methyl-4H-pyran-4-ylidene)malononitrile, 4-(dicyanomethylene)-2-methyl-6-julolidyl-9-enyl-4-H-pyran, 4-(dicyanomethylene)-2-methyl-6-(1,1,7,7-tetramethyljulolidyl-9-enyl)-4H-pyran, 4-(dicyanomethylene)-2-t-butyl-6-(1,1,7,7-tetramethyljulolidin-4-ylvinyl)-4H-pyran, tris(dibenzoylmethane)phenanthroline europium(III), 5,6,11,12-tetraphenylnaphthacene, bis(2-benzo[b]thiophen-2-yl-pyridine)(acetylacetonate) iridium(III), tris(1-phenylisoquinoline) iridium(III), bis(1-phenylisoquinoline)(acetylacetonate) iridium(III), bis [1-(9,9-dimethyl-9H-fluoren-2-yl)isoquinoline] (acetylacetonate) iridium(III), bis[2-(9,9-dimethyl-9H-fluoren-2-yl)quinoline](acetylacetonate) iridium(III), tris[4,4'-di-t-butyl-(2,2')-bipyridine] ruthenium(III)-bis(hexafluorophosphate), tris(2-phenylquinoline) iridium(III), bis(2-phenylquinoline)(acetylacetonate) iridium(III), 2,8-di-t-butyl-5,11-bis (4-t-butylphenyl)-6,12-diphenyltetracene, bis (2-phenylbenzothiazolate) (acetylacetonate) iridium(III), platinum 5,10,15,20-tetraphenyltetrabenzoporphyrin, osmium(II) bis(3-trifluoromethyl-5-(2-pyridine)pyrazolate)dimethylphenylphosphine, osmium(II) bis(3-trifluoromethyl)-5-(4-t-butylpyridyl)-1,2,4-triazolate)diphenylmethyl-phosphine, osmium(II) bis(3-(trifluoromethyl)-5-(2-pyridyl)-1,2,4-triazole)dimethylphenylphosphine, osmium(II) bis(3-(trifluoromethyl)-5-(4-t-butylpyridyl)-1,2,4-triazolate)dimethylphenyl-phosphine, bis[2-(4-n-hexylphenyl)quinoline](acetylacetonate) iridium(III), tris[2-(4-n-hexylphenyl)quinoline] iridium(III), tris[2-phenyl-4-methylquinoline] iridium(III), bis(2-phenylquinoline)(2-(3-methylphenyl)pyridinate)iridium(III), bis(2-(9,9-diethylfluoren-2-yl)-1-phenyl-1H-benzo[d]imidazolato)(acetylacetonate) iridium(III), bis(2-phenylpyridine)(3-(pyridin-2-yl)-2H-chromen-9-onate) iridium(III), bis(2-phenylquinoline)(2,2,6,6-tetramethylheptane-3,5-dionate) iridium(III), bis(phenylisoquinoline)(2,2,6,6-tetramethylheptane-3,5-dionate) iridium(III), iridium(III) bis(4-phenylthieno[3,2-c]pyridinato-N,C²)acetylacetonate, (E)-2-(2-t-butyl-6-(2-(2,6,6-trimethyl-2,4,5,6-tetrahydro-1H-pyrrolo[3,2,1-ij]quinolin-8-yl)vinyl)-4H-pyran-4-ylidene)malononitrile, bis(3-trifluoromethyl-5-(1-isoquinolyl)pyrazolate)(methyldiphenylphosphine) ruthenium, bis [(4-n-hexylphenyl)isoquinoline] (acetylacetonate) iridium(III), platinum(II) octaethylporphin, bis(2-methyldibenzo[f,h]quinoxaline)(acetylacetonate) iridium(III) and tris [(4-n-hexylphenyl)isoquinoline] iridium(III).

Specific examples of electron-transporting layer-forming materials include lithium 8-hydroxyquinolinolate, 2,2',2"-(1,3,5-benzinetriyl)-tris(1-phenyl-1-H-benzimidazole), 2-(4-biphenyl)-5-(4-t-butylphenyl)-1,3,4-oxadiazole, 2,9-dimethyl-4,7-diphenyl-1, 10-phenanthroline, 4,7-diphenyl-1,10-phenanthroline, bis(2-methyl-8-quinolinolate)-4-(phenylphenolato)aluminum, 1,3-bis[2-(2,2'-bipyridin-6-yl)-1,3,4-oxadiazo-5-yl]benzene, 6,6'-bis[5-(biphenyl-4-yl)-1,3,4-oxadiazo-2-yl]-2,2'-bipyridine, 3-(4-biphenyl)-4-phenyl-5-t-butylphenyl-1,2,4-triazole, 4-(naphthalen-1-yl)-3,5-diphenyl-4H-1,2,4-triazole, 2,9-bis(naphthalen-2-yl)-4,7-diphenyl-1,10-phenanthroline, 2,7-bis[2-(2,2'-bipyridin-6-yl)-1,3,4-oxadiazo-5-yl]-9,9-dimethylfluorene, 1,3-bis[2-(4-t-butylphenyl)-1,3,4-oxadiazo-5-yl]benzene, tris(2,4,6-trimethyl-3-(pyridin-3-yl)phenyl)borane, 1-methyl-2-(4-(naphthalen-2-yl)phenyl)-1H-imidazo[4,5f][1,10]phenanthroline, 2-(naphthalen-2-yl)-4,7-diphenyl-1,10-phenanthroline, phenyldipyrenylphosphine oxide, 3,3',5,5'-tetra[(m-pyridyl)-phen-3-yl]biphenyl, 1,3,5-tris[(3-pyridyl)-phen-3-yl]benzene, 4,4'-bis(4,6-diphenyl-1,3,5-triazin-2-yl)biphenyl, 1,3-bis[3,5-di(pyridin-3-yl)phenyl]benzene, bis(10-hydroxybenzo[h]quinolinato)beryllium, diphenylbis(4-(pyridin-3-yl)phenyl)silane and 3,5-di(pyren-1-yl)pyridine.

Examples of electron-injecting layer-forming materials include lithium oxide (Li₂O), magnesium oxide (MgO), alumina (Al₂O₃), lithium fluoride (LiF), sodium fluoride (NaF), magnesium fluoride (MgF₂), cesium fluoride (CsF), strontium fluoride (SrF₂), molybdenum trioxide (MoO₃), aluminum, lithium acetylacetonate (Li(acac)), lithium acetate and lithium benzoate.

Examples of cathode materials include aluminum, magnesium-silver alloys, aluminum-lithium alloys, lithium, sodium, potassium and cesium.

In cases where a thin film obtained from the charge-transporting varnish of the invention serves as the hole-injecting layer, another example of a method of fabricating the organic EL device of the invention is as follows.

In the above-described organic EL device production process, instead of carrying out vacuum evaporation operations for a hole-transporting layer, a light-emitting layer, an electron-transporting layer and an electron-injecting layer, by successively forming a hole-transporting layer and a light-emitting layer, it is possible to produce an organic EL device having a charge-transporting thin film formed using the charge-transporting varnish of the invention. Specifically, the charge-transporting varnish of the invention is applied onto an anode substrate, thus forming a hole-injecting layer by the above-described method. A hole-transporting layer and a light-emitting layer are successively formed thereon, and a cathode material is vapor-deposited on top, thereby giving an organic EL device.

The cathode and anode materials used here may be similar to those described above, and similar cleaning treatment and surface treatment may be carried out.

The method of forming the hole-transporting layer and the light-emitting layer is exemplified by a film-forming method that involves adding a solvent to a hole-transporting polymer material or a light-emitting polymer material, or to the material obtained by adding a dopant to these, thereby dissolving or uniformly dispersing the material, and then applying the resulting solution or dispersion onto, respectively, the hole-injecting layer or the hole-transporting layer and subsequently baking the applied layer.

Examples of hole-transporting polymer materials include poly[(9,9-dihexylfluorenyl-2,7-diyl)-co-(N,N'-bis{p-butylphenyl}-1,4-diaminophenylene)], poly[{9,9-dioctylfluorenyl-2,7-diyl)-co-(N,N'-bis{p-buty}phenyl}-1,1'-biphenylene-4,4-diamine)], poly[(9,9-bis{1'-penten-5'-yl}fluorenyl-2,7-diyl)-co-(N,N'-bis{p-butylphenyl}-1,4-diaminophenylene)], poly[N,N'-bis(4-butylphenyl)-N,N'-bis(phenyl)benzidine] end-capped with polysilsesquioxane and poly[(9,9-dioctylfluorenyl-2,7-diyl)-co-(4,4'-(N-(p-butylphenyl))diphenylamine)].

Examples of light-emitting polymer materials include polyfluorene derivatives such as poly(9,9-dialkylfluorene) (PDAF), poly(phenylene vinylene) derivatives such as poly(2-methoxy-5-(2'-ethylhexoxy)-1,4-phenylene vinylene) (MEH-PPV), polythiophene derivatives such as poly(3-alkylthiophene) (PAT), and polyvinylcarbazole (PVCz).

Examples of the solvent include toluene, xylene and chloroform. Examples of the method of dissolution or uniform dispersion include stirring, stirring under applied heat, and ultrasonic dispersion.

Examples of the coating method include, but are not particularly limited to, inkjet printing, spraying, dipping, spin coating, transfer printing, roll coating and brush coating. Coating is preferably carried out in an inert gas atmosphere such as nitrogen or argon.

Examples of the baking method include methods that involve heating in an oven or on a hot plate, either within an inert gas atmosphere or in a vacuum.

An example is described below of a method of fabricating the organic EL device of the invention in a case where a thin film obtained from the charge-transporting varnish of the invention serves as a hole-injecting-and-transporting layer.

A hole-injecting-and-transporting layer is formed on an anode substrate, and a light-emitting layer, an electron-transporting layer, an electron-injecting layer and a cathode are provided in this order on the hole-injecting-and-transporting layer. Methods of forming the light-emitting layer, electron-transporting layer and electron-injecting layer, and illustrative examples of each, are exemplified in the same way as above.

The anode material, the light-emitting layer, the light-emitting dopant, the materials which form the electron-transporting layer and the electron-blocking layer, and the cathode material are exemplified in the same way as above.

A hole-blocking layer, an electron-blocking layer or the like may be optionally provided between the electrodes and any of the above layers. By way of illustration, an example of a material that forms an electron-blocking layer is tris(phenylpyrazole)iridium.

The materials which make up the layers that form the anode, the cathode and the layers formed therebetween differ according to whether a device provided with a bottom emission structure or a top emission structure is to be fabricated, and so are suitably selected while taking this into account.

Generally, in a device having a bottom emission structure, a transparent anode is used on the substrate side and light is extracted from the substrate side, whereas in a device having a top emission structure, a reflective anode made of metal is used and light is extracted from a transparent electrode (cathode) in the opposite direction from the substrate. Accordingly, with regard to, for example, the anode material, when fabricating a device having a bottom emission structure, a transparent anode of (ITO) or the like is used, and when fabricating a device having a top emission structure, a reflective anode of Al/Nd or the like is used.

To prevent a deterioration in device characteristics, the organic EL device of the invention may be sealed in the usual manner with, if necessary, a desiccant or the like.

### EXAMPLES

Working Examples are given below to more concretely illustrate the invention, although the invention is not limited by these Examples. The equipment used was as follows.

| | |
|---|---|
| (1) ¹H-NMR Measurement: | ECX-300, from JEOL Ltd. |
| (2) ES-MS: | ZQ 2000, from Waters Corporation |
| (3) MALDI-TOF-MS: | Autoflex III SmartBeam, from Bruker Daltonics |
| (4) Substrate Cleaning: | Substrate cleaning machine (reduced-pressure plasma system), from Choshu Industry Co., Ltd. |
| (5) Varnish Coating: | MS-A100 Spin Coater, from Mikasa Co., Ltd. |
| (6) Film Thickness Measurement: | Surfcorder ET-4000 microfigure measuring instrument, from Kosaka Laboratory, Ltd. |
| (7) Organic EL Device Fabrication: | C-E2L1G1-N Multifunction Vapor Deposition System, from Choshu Industry Co., Ltd. |
| (8) Measurement of Brightness, etc. of Organic EL Device: | I-V-L Measurement System from Tech World, Inc. |

### [1] Compound Synthesis

### [Synthesis Example 1] Synthesis of Reference Compound H1

Reference Compound H1 of the formula shown below was synthesized according to the method described in JP-A 2013-2324169.

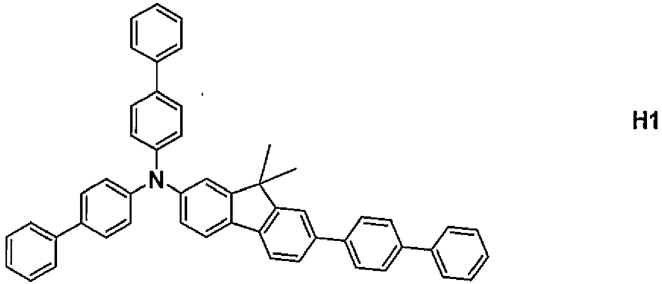

### [Synthesis Example 2] Synthesis of Intermediate A

A flask was charged with 7.42 g of 2-bromo-7-iodofluorene, 30 mL of dimethylsulfoxide, 330 mg of potassium iodide and 2.81 g of potassium hydroxide, following which 30 mL of a mixture of diethylene glycol 2-bromoethyl methyl ether (concentration, 330 g/L) in dimethylsulfoxide was added dropwise while stirring the flask contents on an ice bath. After 1.5 hours, the ice bath was removed and stirring was continued for another 58.5 hours at room temperature. Deionized water and ethyl acetate were added to the reaction mixture, and separatory treatment was carried out. The resulting organic layer was washed with deionized water and saturated saline, and dried over magnesium sulfate. The solvent was driven off under reduced pressure, after which separation and purification were carried out by silica gel column chromatography (developing solvent: hexane/ethyl acetate = 40/60 → 0/100). The fractions containing the target substance were combined and the solvent was driven off under reduced pressure, following which drying was carried out, giving 8.16 g (yield, 62 %) of Intermediate A of the formula shown below. The results of ¹H-NMR and ES-MS measurement are shown below.
¹H-NMR (300 MHz, CDCl₃) δ [ppm]:
7.74 (d, J = 1.4 Hz, 1H), 7.67 (dd, J = 8.2, 1.4 Hz, 1H),
7.44-7.54 (m, 3H), 7.39 (d, J = 8.2 Hz, 1H), 3.47-3.55 (m, 8H),
3.37-3.41 (m, 4H), 3.34 (s, 6H), 3.19-3.22 (m, 4H),
2.79 (t, J = 7.2 Hz, 4H), 2.33 (t, J = 7.2 Hz, 4H).
ES(+)-MS, m/Z; found: 680.19 ([M+NH₄]⁺ calculated: 680.11).

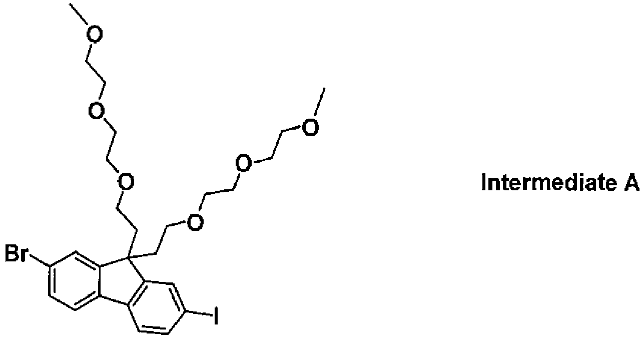

### [Synthesis Example 3] Synthesis of Intermediate B

A flask was charged with 4.97 g of Intermediate A, 1.49 g of 4-biphenylboric acid, 175 mg of Pd(PPh₃)₄, 35 mL of toluene and 7 mL of an aqueous solution of sodium carbonate (concentration, 210 g/L), and the flask contents were stirred for 4 hours under refluxing. After allowing the system to cool, deionized water and ethyl acetate were added to the reaction mixture and separatory treatment was carried out. The resulting organic layer was washed with deionized water and saturated saline, and dried over magnesium sulfate. The solvent was driven off under reduced pressure, after which separation and purification were carried out by silica gel column chromatography (developing solvent: hexane/ethyl acetate = 40/60 → 20/80). The fractions containing the target substance were combined and the solvent was driven off under reduced pressure, following which drying was carried out, giving 2.08 g (yield, 40 %) of Intermediate B of the formula shown below. The results of ¹H-NMR and ES-MS measurement are shown below.
¹H-NMR (300 MHz, CDCl₃) δ [ppm]:
7.32-7.75 (m, 15H), 3.42-3.51 (m, 8H), 3.36-3.40 (m, 4H),
3.30 (s, 6H), 3.20-3.23 (m, 4H), 2.82-2.88 (m, 4H),
2.39-2.45 (m, 4H)
ES(+)-MS, m/Z; found: 706.46 ([M+NH₄]⁺ calculated: 706.27).

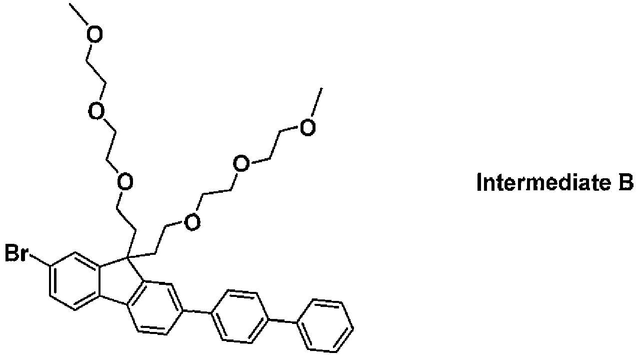

### [Synthesis Example 4] Synthesis of Compound TEG-H1

A flask was charged with 2.08 g of Intermediate B, 1.06 g of bis(4-biphenylyl)amine, 51.8 mg of Pd(dba)₂ and 397 mg of sodium tert-butoxide, and the system was flushed with nitrogen. Next, 15 mL of toluene and 0.5 mL of a toluene solution of tri-t-butylphosphine that was prepared beforehand (concentration, 73 g/L) were added and the flask contents were stirred for 2 hours at 80°C. After allowing the system to cool, deionized water and ethyl acetate were added to the reaction mixture and separatory treatment was carried out. The resulting organic layer was washed with deionized water and saturated saline, and dried over magnesium sulfate. The solvent was driven off under reduced pressure, after which separation and purification were carried out by silica gel column chromatography (developing solvent: hexane/ethyl acetate = 70/30 → 0/100). The fractions containing the target substance were combined and the solvent was driven off under reduced pressure, following which drying was carried out, giving 2.19 g (yield, 78 %) of compound TEG-H1 of the formula shown below. The results of ¹H-NMR and MALDI-TOF MS measurement are shown below.
¹H-NMR (300 MHz, CDCl₃) δ [ppm]:
7.14-7.76 (m, 33H), 3.41-3.52 (m, 12H),
3.25-3.35 (m, 10H), 2.86-2.99 (m, 4H), 2.26-2.43 (m, 4H)
MALDI-TOF MS, m/Z; found: 929.07 ([m]⁺ calculated: 929.47).

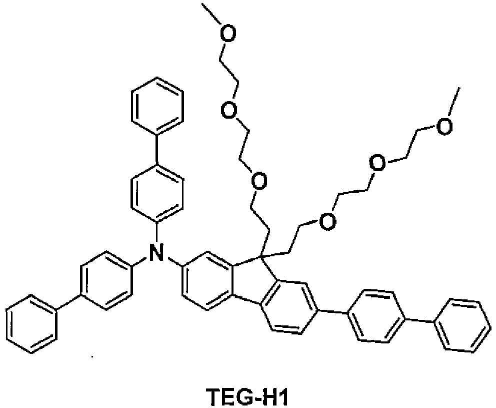

### [2] Preparation of Charge-Transporting Varnish

### [Working Example 1-1]

A charge-transporting varnish was obtained by dissolving 0.052 g of the compound TEG-H1 and 0.010 g of phosphotungstic acid (Japan New Metals Co., Ltd.) in 2 g of DMIB under a nitrogen atmosphere, and using a PTFE filter having a pore size of 0.2 µm to filter the resulting solution.

### [Reference Example 1-2]

A charge-transporting varnish was obtained by dissolving 0.025 g of the reference compound H1 and 0.005 g of phosphotungstic acid (Japan New Metals Co., Ltd.) in 2 g of DMIB under a nitrogen atmosphere, and using a PTFE filter having a pore size of 0.2 µm to filter the resulting solution.

### [Comparative Example 1]

A charge-transporting varnish was obtained by dissolving 0.062 g of the compound TEG-H1 in 2 g of DMIB under a nitrogen atmosphere, and using a PTFE filter having a pore size of 0.2 µm to filter the resulting solution.

### [3] Evaluation of Organic EL Device

### [Working Example 2-1]

The varnish obtained in Working Example 1-1 was coated onto an ITO substrate using a spin coater, subsequently dried for 1 minute at 80°C, and then baked for 15 minutes at 230°C in open air, thereby forming a uniform 25 nm thin film (hole-injecting layer) on an ITO substrate. A glass substrate with dimensions of 25 mm x 25 mm × 0.7 mm (t) and having indium-tin oxide (ITO) patterned on the surface to a film thickness of 150 nm was used as the ITO substrate. Prior to use, impurities on the surface were removed with an O₂ plasma cleaning system (150 W, 30 seconds).

A 30 nm film of α-NPD was formed thereon using a vapor deposition system (degree of vacuum, 1.0×10⁻⁵ Pa). The deposition rate at this time was set to 0.2 nm/s. CBP and Ir(PPy)₃ were subsequently co-deposited. Co-deposition was carried out to a thickness of 40 nm while controlling the deposition rate so that the Ir(PPy)₃ concentration becomes 6 %. Next, thin films of BAlq, lithium fluoride and aluminum were successively deposited, giving an organic EL device. At this time, vapor deposition was carried out at a rate of 0.2 nm/s for BAlq and aluminum, and at a rate of 0.02 nm/s for lithium fluoride. The film thicknesses were set to, respectively, 20 nm, 0.5 nm and 100 nm.

To prevent the device characteristics from deteriorating due to the influence of oxygen, moisture and the like in air, the organic EL device was sealed with sealing substrates, following which the device characteristics were evaluated. Sealing was carried out by the following procedure.

The organic EL device was placed between sealing substrates in a nitrogen atmosphere having an oxygen concentration of not more than 2 ppm and a dew point of not more than -85°C, and the sealing substrates were laminated together using an adhesive (MORESCO Moisture Cut WB90US(P), from Moresco Corporation). At this time, a desiccant (HD-071010W-40, from Dynic Corporation) was placed, together with the organic EL device, within the sealing substrates. The laminated sealing substrates were irradiated with UV light (wavelength, 365 nm; dosage, 6,000 mJ/cm²) and then annealed at 80°C for 1 hour to cure the adhesive.

### [Reference Example 2-2]

Aside from using the varnish obtained in Reference Example 1-2 instead of the varnish obtained in Working Example 1-1, an organic EL device was fabricated in the same way as in Working Example 2-1.

### [Comparative Example 2]

Aside from using the varnish obtained in Comparative Example 1 instead of the varnish obtained in Working Example 1-1, an organic EL device was fabricated in the same way as in Working Example 2-1.

Table 1 shows the current density, brightness, current efficiency and brightness half-life (initial brightness, 5,000 cd/m²) at a driving voltage of 10V.

**[Table 1]**

| | Current density (mA/cm²) | Brightness (cd/m²) | Current efficiency (cd/A) | Brightness half-life (h) |
|---|---|---|---|---|
| Working Example 2-1 | 10 | 2,780 | 29.2 | 520 |
| Reference Example 2-2 | 11 | 2,950 | 26.9 | 490 |
| Comparative Example 2 | 2 | 9 | 0.6 | - |

As shown in Table 1, when charge-transporting thin films obtained from charge-transporting varnishes of the invention were used as the hole-injecting layer, organic EL devices having excellent brightness characteristics and durability were obtained.

## Claims

1. A charge-transporting varnish comprising a charge-transporting substance consisting of the arylamine derivative of formula (1) below, a dopant, and an organic solvent 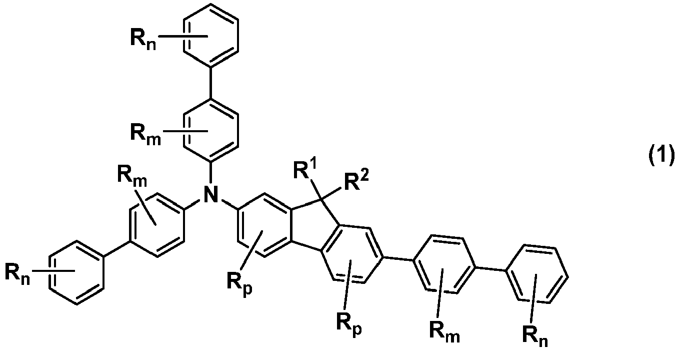
wherein R¹ and R² are each independently an alkyl group of 2 to 20 carbon atoms which includes at least one ether structure, and some or all of the hydrogen atoms bonded to carbon atoms on said groups may be substituted with halogen atoms;
each R is independently a halogen atom, a nitro group, a cyano group, an alkyl group of 1 to 20 carbon atoms which may be substituted with Z¹, an alkenyl group of 2 to 20 carbon atoms which may be substituted with Z¹, an alkynyl group of 2 to 20 carbon atoms which may be substituted with Z¹, an alkoxy group of 1 to 20 carbon atoms which may be substituted with Z¹, an alkenyloxy group of 2 to 20 carbon atoms which may be substituted with Z¹, or an alkynyloxy group of 2 to 20 carbon atoms which may be substituted with Z¹;
Z¹ is a halogen atom, a nitro group, a cyano group, an aryl group of 6 to 20 carbon atoms which may be substituted with Z², a heteroaryl group of 2 to 20 carbon atoms which may be substituted with Z², an alkoxy group of 1 to 20 carbon atoms which may be substituted with Z², an alkenyloxy group of 2 to 20 carbon atoms which may be substituted with Z², an alkynyloxy group of 2 to 20 carbon atoms which may be substituted with Z², an aryl group of 6 to 20 carbon atoms which may be substituted with Z², or a heteroaryl group of 2 to 20 carbon atoms which may be substituted with Z²;
Z² is a halogen atom, a nitro group or a cyano group; and
each subscript n is independently an integer from 0 to 5, each subscript m is independently an integer from 0 to 4, and each subscript p is independently an integer from 0 to 3.

2. The charge-transporting varnish of claim 1, wherein the subscripts n, m and p are all 0.

3. The charge-transporting varnish of any one of claims 1 or 2, wherein the dopant is a heteropolyacid.

4. A charge-transporting thin film comprising a charge-transporting substance consisting of the arylamine derivative of formula (1) of claim 1 and a dopant according to claim 1.

5. An organic electroluminescent device comprising the charge-transporting thin film of claim 4.

6. The organic electroluminescent device of claim 5, wherein the charge-transporting thin film is a hole-injecting layer.

7. An arylamine derivative of formula (1') 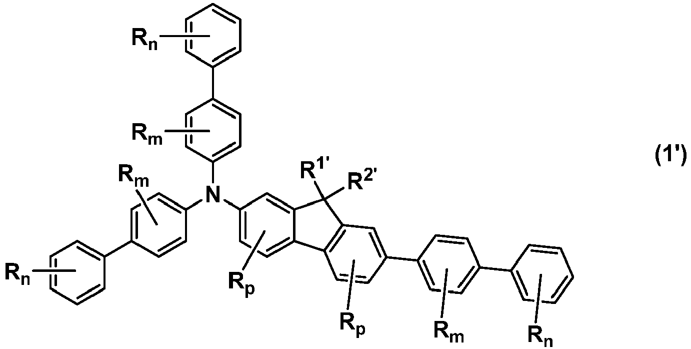
wherein R¹ and R^{2'} are each independently an alkyl group of 2 to 20 carbon atoms which includes at least one ether structure, and some or all of the hydrogen atoms bonded to carbon atoms on said groups may be substituted with halogen atoms;
each R is independently a halogen atom, a nitro group, a cyano group, an alkyl group of 1 to 20 carbon atoms which may be substituted with Z¹, an alkenyl group of 2 to 20 carbon atoms which may be substituted with Z¹, an alkynyl group of 2 to 20 carbon atoms which may be substituted with Z¹, an alkoxy group of 1 to 20 carbon atoms which may be substituted with Z², an alkenyloxy group of 2 to 20 carbon atoms which may be substituted with Z¹, or an alkynyloxy group of 2 to 20 carbon atoms which may be substituted with Z¹;
Z¹ is a halogen atom, a nitro group, a cyano group, an aryl group of 6 to 20 carbon atoms which may be substituted with Z², a heteroaryl group of 2 to 20 carbon atoms which may be substituted with Z², an alkoxy group of 1 to 20 carbon atoms which may be substituted with Z², an alkenyloxy group of 2 to 20 carbon atoms which may be substituted with Z², an alkynyloxy group of 2 to 20 carbon atoms which may be substituted with Z², an aryl group of 6 to 20 carbon atoms which may be substituted with Z², or a heteroaryl group of 2 to 20 carbon atoms which may be substituted with Z²;
Z² is a halogen atom, a nitro group or a cyano group; and
each subscript n is independently an integer from 0 to 5, each subscript m is independently an integer from 0 to 4, and each subscript p is independently an integer from 0 to 3.

## Patentansprüche

1. Ladungstransportlack, umfassend eine Ladungstransportsubstanz, die aus einem Arylaminderivat der nachstehenden Formel (1), einem Dotiermittel und einem organischen Lösungsmittel besteht: 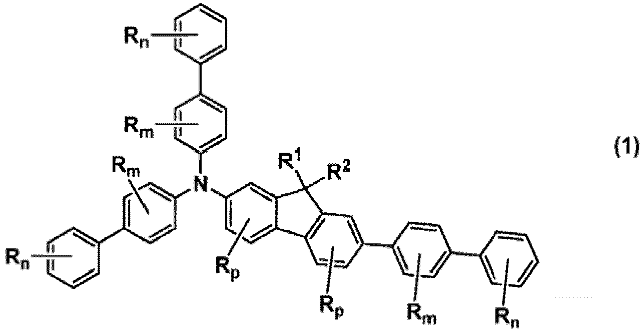
worin R¹ und R² jeweils unabhängig eine Alkylgruppe mit 2 bis 20 Kohlenstoffatomen sind, die zumindest eine Etherstruktur aufweist, und worin manche oder alle der Wasserstoffatome, die an Kohlenstoffatome auf den Gruppen gebunden sind, durch Halogenatome ersetzt sein können;
die R jeweils unabhängig ein Halogenatom, eine Nitrogruppe, eine Cyanogruppe, eine gegebenenfalls mit Z¹ substituierte Alkylgruppe mit 1 bis 20 Kohlenstoffatomen, eine gegebenenfalls mit Z¹ substituierte Alkenylgruppe mit 2 bis 20 Kohlenstoffatomen, eine gegebenenfalls mit Z¹ substituierte Alkinylgruppe mit 2 bis 20 Kohlenstoffatomen, eine gegebenenfalls mit Z¹ substituierte Alkoxygruppe mit 1 bis 20 Kohlenstoffatomen, eine gegebenenfalls mit Z¹ substituierte Alkenyloxygruppe mit 2 bis 20 Kohlenstoffatomen oder eine gegebenenfalls mit Z¹ substituierte Alkinyloxygruppe mit 2 bis 20 Kohlenstoffatomen sind;
Z¹ ein Halogenatom, eine Nitrogruppe, eine Cyanogruppe, eine gegebenenfalls mit Z² substituierte Arylgruppe mit 6 bis 20 Kohlenstoffatomen, eine gegebenenfalls mit Z² substituierte Heteroarylgruppe mit 2 bis 20 Kohlenstoffatomen, eine gegebenenfalls mit Z² substituierte Alkoxygruppe mit 1 bis 20 Kohlenstoffatomen, eine gegebenenfalls mit Z² substituierte Alkenyloxygruppe mit 2 bis 20 Kohlenstoffatomen, eine gegebenenfalls mit Z² substituierte Alkinyloxygruppe mit 2 bis 20 Kohlenstoffatomen, eine gegebenenfalls mit Z² substituierte Arylgruppe mit 6 bis 20 Kohlenstoffatomen oder eine gegebenenfalls mit Z² substituierte Heteroarylgruppe mit 2 bis 20 Kohlenstoffatomen ist;
Z² ein Halogenatom, eine Nitrogruppe oder eine Cyanogruppe ist; und
die Indices n jeweils unabhängig eine ganze Zahl von 0 bis 5 sind, die Indices m jeweils unabhängig eine ganze Zahl von 0 bis 4 sind und die Indices p jeweils unabhängig eine ganze Zahl von 0 bis 3 sind.

2. Ladungstransportlack nach Anspruch 1, wobei die Indices n, m und p allesamt 0 sind.

3. Ladungstransportlack nach einem der Ansprüche 1 oder 2, wobei das Dotiermittel eine Heteropolysäure ist.

4. Ladungstransport-Dünnfilm, der eine Ladungstransportsubstanz umfasst, die aus einem Arylaminderivat der Formel (1) nach Anspruch 1 und einem Dotiermittel nach Anspruch 1 besteht.

5. Organische elektrolumineszierende Vorrichtung, die einen Ladungstransport-Dünnfilm nach Anspruch 4 umfasst.

6. Organische elektrolumineszierende Vorrichtung nach Anspruch 5, wobei der Ladungstransport-Dünnfilm eine Loch-Injektionsschicht ist.

7. Arylaminderviat der Formel (1') 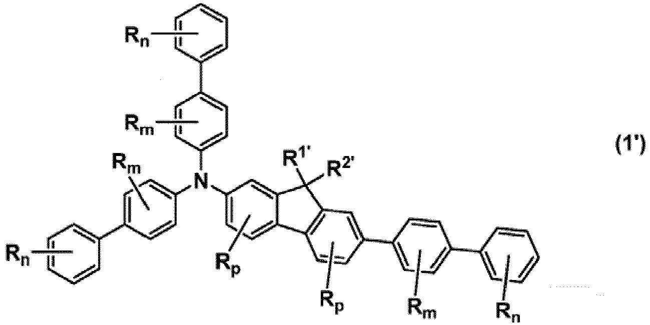
worin R¹' und R^{2'} jeweils unabhängig eine Alkylgruppe mit 2 bis 20 Kohlenstoffatomen sind, die zumindest eine Etherstruktur aufweist, und worin manche oder alle der an Kohlenstoffatome auf den Gruppen gebundenen Wasserstoffatome durch Halogenatome ersetzt sein können;
die R jeweils unabhängig ein Halogenatom, eine Nitrogruppe, eine Cyanogruppe, eine gegebenenfalls mit Z¹ substituierte Alkylgruppe mit 1 bis 20 Kohlenstoffatomen, eine gegebenenfalls mit Z¹ substituierte Alkenylgruppe mit 2 bis 20 Kohlenstoffatomen, eine gegebenenfalls mit Z¹ substituierte Alkinylgruppe mit 2 bis 20 Kohlenstoffatomen, eine gegebenenfalls mit Z¹ substituierte Alkoxygruppe mit 1 bis 20 Kohlenstoffatomen, eine gegebenenfalls mit Z¹ substituierte Alkenyloxygruppe mit 2 bis 20 Kohlenstoffatomen oder eine gegebenenfalls mit Z¹ substituierte Alkinyloxygruppe mit 2 bis 20 Kohlenstoffatomen sind;
Z¹ ein Halogenatom, eine Nitrogruppe, eine Cyanogruppe, eine gegebenenfalls mit Z² substituierte Arylgruppe mit 6 bis 20 Kohlenstoffatomen, eine gegebenenfalls mit Z² substituierte Heteroarylgruppe mit 2 bis 20 Kohlenstoffatomen, eine gegebenenfalls mit Z² substituierte Alkoxygruppe mit 1 bis 20 Kohlenstoffatomen, eine gegebenenfalls mit Z² substituierte Alkenyloxygruppe mit 2 bis 20 Kohlenstoffatomen, eine gegebenenfalls mit Z² substituierte Alkinyloxygruppe mit 2 bis 20 Kohlenstoffatomen, eine gegebenenfalls mit Z² substituierte Arylgruppe mit 5 bis 20 Kohlenstoffatomen oder eine gegebenenfalls mit Z² substituierte Heterorarylgruppe mit 2 bis 20 Kohlenstoffatomen ist;
Z² ein Halogenatom, eine Nitrogruppe oder eine Cyanogruppe ist; und
die Indices n jeweils unabhängig eine ganze Zahl von 0 bis 5 sind, die Indices m jeweils unabhängig eine ganze Zahl von 0 bis 4 sind und die Indices p jeweils unabhängig eine ganze Zahl von 0 bis 3 sind.

## Revendications

1. Vernis de transport de charge comprenant une substance de transport de charge consistant en le dérivé d'arylamine de formule (1) ci-dessous, un dopant, et un solvant organique 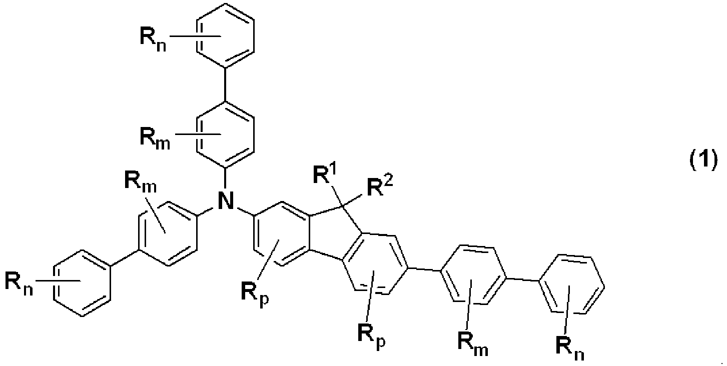 formule dans laquelle
chacun de R¹ et R² est indépendamment un groupe alkyle de 2 à 20 atomes de carbone qui comprend au moins une structure d'éther, et tout ou partie des atomes d'hydrogène liés aux atomes de carbone sur lesdits groupes peuvent être remplacés par des atomes d'halogène ;
chaque R est indépendamment un atome d'halogène, un groupe nitro, un groupe cyano, un groupe alkyle de 1 à 20 atomes de carbone qui peut être substitué par Z¹, un groupe alcényle de 2 à 20 atomes de carbone qui peut être substitué par Z¹, un groupe alcynyle de 2 à 20 atomes de carbone qui peut être substitué par Z¹, un groupe alcoxy de 1 à 20 atomes de carbone qui peut être substitué par Z¹, un groupe alcényloxy de 2 à 20 atomes de carbone qui peut être substitué par Z¹, ou un groupe alcynyloxy de 2 à 20 atomes de carbone qui peut être substitué par Z¹ ;
Z¹ est un atome d'halogène, un groupe nitro, un groupe cyano, un groupe aryle de 6 à 20 atomes de carbone qui peut être substitué par Z², un groupe hétéroaryle de 2 à 20 atomes de carbone qui peut être substitué par Z², un groupe alcoxy de 1 à 20 atomes de carbone qui peut être substitué par Z², un groupe alcényloxy de 2 à 20 atomes de carbone qui peut être substitué par Z², un groupe alcynyloxy de 2 à 20 atomes de carbone qui peut être substitué par Z², un groupe aryle de 6 à 20 atomes de carbone qui peut être substitué par Z², ou un groupe hétéroaryle de 2 à 20 atomes de carbone qui peut être substitué par Z² ;
Z² est un atome d'halogène, un groupe nitro ou un groupe cyano ; et
chaque indice n est indépendamment un entier de 0 à 5, chaque indice m est indépendamment un entier de 0 à 4, et chaque indice p est indépendamment un entier de 0 à 3.

2. Vernis de transport de charge selon la revendication 1, dans lequel les indices n, m et p valent tous 0.

3. Vernis de transport de charge selon l'une quelconque des revendications 1 et 2, dans lequel le dopant est un hétéropolyacide.

4. Film mince de transport de charge comprenant une substance de transport de charge consistant en le dérivé d'arylamine de formule (1) de la revendication 1 et un dopant selon la revendication 1.

5. Dispositif électroluminescent organique comprenant le film mince de transport de charge de la revendication 4.

6. Dispositif électroluminescent organique selon la revendication 5, dans lequel le film mince de transport de charge est une couche d'injection de trou.

7. Dérivé d'arylamine de formule (1') 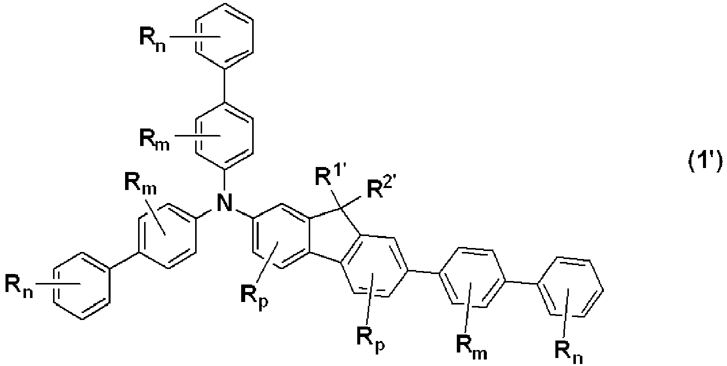 formule dans laquelle
chacun de R^{1'} et R^{2'} est indépendamment un groupe alkyle de 2 à 20 atomes de carbone qui comprend au moins une structure d'éther, et tout ou partie des atomes d'hydrogène liés aux atomes de carbone sur lesdits groupes peuvent être remplacés par des atomes d'halogène ;
chaque R est indépendamment un atome d'halogène, un groupe nitro, un groupe cyano, un groupe alkyle de 1 à 20 atomes de carbone qui peut être substitué par Z¹, un groupe alcényle de 2 à 20 atomes de carbone qui peut être substitué par Z¹, un groupe alcynyle de 2 à 20 atomes de carbone qui peut être substitué par Z¹, un groupe alcoxy de 1 à 20 atomes de carbone qui peut être substitué par Z¹, un groupe alcényloxy de 2 à 20 atomes de carbone qui peut être substitué par Z¹, ou un groupe alcynyloxy de 2 à 20 atomes de carbone qui peut être substitué par Z¹ ;
Z¹ est un atome d'halogène, un groupe nitro, un groupe cyano, un groupe aryle de 6 à 20 atomes de carbone qui peut être substitué par Z², un groupe hétéroaryle de 2 à 20 atomes de carbone qui peut être substitué par Z², un groupe alcoxy de 1 à 20 atomes de carbone qui peut être substitué par Z², un groupe alcényloxy de 2 à 20 atomes de carbone qui peut être substitué par Z², un groupe alcynyloxy de 2 à 20 atomes de carbone qui peut être substitué par Z², un groupe aryle de 6 à 20 atomes de carbone qui peut être substitué par Z², ou un groupe hétéroaryle de 2 à 20 atomes de carbone qui peut être substitué par Z² ;
Z² est un atome d'halogène, un groupe nitro ou un groupe cyano ; et
chaque indice n est indépendamment un entier de 0 à 5, chaque indice m est indépendamment un entier de 0 à 4, et chaque indice p est indépendamment un entier de 0 à 3.
